# EUROPÄISCHE PATENTANMELDUNG

(11) **EP 4 124 631 A1**
(43) Veröffentlichungstag der Anmeldung: **01.02.2023**
(21) Anmeldenummer: 21188304.6
(22) Anmeldetag: 28.07.2021
(51) Int. Cl.: C08G 59/50

(54) **AMINHÄRTER MIT HOHEM ERNEUERBAREM KOHLENSTOFFANTEIL**

(71) Anmelder: Sika Technology AG, 6340 Baar (CH)
(72) Erfinder: KASEMI, Edis, 8046 Zürich (CH); STADELMANN, Ursula, 8046 Zürich (CH); KRAMER, Andreas, 8008 Zürich (CH); BURCKHARDT, Urs, 8049 Zürich (CH)
(74) Vertreter: Sika Patent Attorneys

(57) **Zusammenfassung**

Gegenstand der Erfindung ist die Verwendung eines Härters enthaltend mindestens ein Amin der Formel (I) zum Aushärten von Epoxidharzen.

Das Amin der Formel (I) verfügt über einen hohen Renewable Carbon Index (RCI) und ermöglicht besonders nachhaltige Epoxidharz-Produkte. Es ist überraschend einfach herstellbar, geruchsarm, verdünnt das Epoxidharz besonders gut und ermöglicht eine schnelle und störungsfreie Aushärtung mit überraschend geringer Exotherm ie.

## Beschreibung

### Technisches Gebiet

Die Erfindung betrifft alkylierte Amine mit einem hohen erneuerbaren Kohlenstoffanteil und ihre Verwendung als Härter für Epoxidharze.

### Stand der Technik

Amine werden in der Industrie und im Bauwesen unter anderem als Härter in Epoxidharz-Zusammensetzungen verwendet. Dabei sind je nach Anwendung Eigenschaften gefragt wie eine hohe Reaktivität bei wenig Exothermie und/oder eine schnelle, störungsfreie Aushärtung bei Umgebungstemperaturen zu Beschichtungen oder Körpern mit ebenmässiger Oberfläche ohne Blushing-bedingte Trübungen, Flecken oder Krater. Die ausgehärteten Körper oder Beschichtungen sollen eine hohe Härte bei geringer Sprödigkeit besitzen, um mechanischer Beanspruchung möglichst gut zu widerstehen, und für optisch anspruchsvolle Anwendungen über einen hohen Glanzgrad und eine geringe Neigung zum Vergilben verfügen. Dabei ist für viele Anwendungen entscheidend, dass die Epoxidharz-Zusammensetzung eine niedrige Viskosität aufweist, damit sie möglichst einfach und schnell appliziert werden kann, gut verläuft und entlüftet und gegebenenfalls gut in die Substrate eindringt. Da viele Epoxidharze eine eher hohe Viskosität aufweisen, ist eine effiziente Verdünnung derselben durch die Aminhärter besonders vorteilhaft, weil zur Einstellung einer geeigneten Viskosität weniger Verdünner oder Lösemittel eingesetzt werden müssen und/oder ein höherer Füllgrad ermöglicht wird.

Heutzutage werden vermehrt Epoxidharz-Zusammensetzungen nachgefragt, welche nachhaltig sind. Insbesondere sollen sie einen hohen Gehalt an Rohstoffen aus erneuerbaren biologischen Quellen enthalten, also zu einem hohen Anteil biobasiert sein. Es besteht deshalb ein Bedarf nach nachhaltigen Aminhärtern. Ein gebräuchliches Mass für die Nachhaltigkeit von chemischen Rohstoffen ist der Renewable Carbon Index (RCI), welcher den Kohlenstoffanteil aus erneuerbaren biologischen Quellen angibt. Er ergibt sich durch Division der Anzahl Kohlenstoffatome abgeleitet von erneuerbaren Quellen mit der gesamten Anzahl Kohlenstoffatome im Rohstoff.

Härter enthaltend Amine mit hohem Renewable Carbon Index sind bekannt, beispielsweise aus US 9,676,899 oder WO 2015/124792, wo Bis(aminomethyl)-furane bzw. Bisfurfurylamine und deren Verwendung als Härter für Epoxidharze beschrieben sind. Diese Amine sind aber aufwändig in der Herstellung, anfällig auf Blushing und ihre Verdünnungswirkung auf das Epoxidharz ist verbesserungsfähig.

Aus EP 3,350,245 sind nachhaltige Härter enthaltend alkylierte Amine mit einem Tetrahydrofuranring bekannt. Diese Härter zeigen eine unvollständige Aushärtung mit reduzierter Endhärte, vor allem bei flächiger Anwendung und unter kalten Bedingungen wie beispielsweise 8 °C.

Weiterhin bekannt sind benzylierte Amine, beispielsweise aus EP 2,731,927, EP 3,180,383 oder EP 3,344,677. Als Härter für Epoxidharze haben diese Amine eine gute Verdünnungswirkung und ermöglichen eine schnelle, störungsfreie Aushärtung, auch bei flächiger Anwendung und tiefen Umgebungstemperaturen. Sie sind aber nicht biobasiert herstellbar.

### Darstellung der Erfindung

Aufgabe der vorliegenden Erfindung ist es daher, einen Härter für Epoxidharze zur Verfügung zu stellen, welcher einen hohen Renewable Carbon Index (RCI) aufweist, einfach herstellbar ist, das Epoxidharz gut verdünnt und eine störungsfreie Aushärtung ermöglicht.

Überraschenderweise wird diese Aufgabe mit einem Härter enthaltend ein Amin der Formel (I) wie in Anspruch 1 beschrieben gelöst. Das Amin der Formel (I) wird in einem einfachen Prozess erhalten aus der reduktiven Alkylierung eines primären aliphatischen Amins mit auf nachwachsenden Rohstoffen basierendem Furfural. Das Amin der Formel (I) verfügt über einen hohen RCI, bevorzugt mindestens 0.45, insbesondere mindestens 0.7.

Der Härter enthaltend das Amin der Formel (I) vermag Epoxidharze überraschend stark zu verdünnen. Insbesondere mit N-Furfuryl-1,2-ethandiamin wird das Epoxidharz besonders effizient verdünnt, sogar stärker als mit dem bekannten N-Benzyl-1,2-ethandiamin. Dies ist überraschend, da die zum Aushärten des Epoxidharzes benötigte Einsatzmenge an N-Furfuryl-1,2-ethandiamin aufgrund des niedrigeren Amin-Equivalenzgewichts niedriger ist als bei N-Benzyl-1,2-ethandiamin und man aufgrund des Sauerstoffs im Furanring und somit der Möglichkeit zur Wasserstoffbrückenbildung einen geringeren verdünnenden Effekt als bei N-Benzyl-1,2-ethandiamin erwarten würde. Der erfindungsgemässe Härter ist sehr geruchsarm, was für viele Anwendungen ein weiterer grosser Vorteil ist. Er ermöglicht eine schnelle und störungsfreie Aushärtung zu hoher Endhärte. Besonders überraschend ist die geringe Exothermie bei der Aushärtung, welche deutlich geringer ist als bei der Verwendung von N-Benzyl-1,2-ethandiamin, wobei die Verarbeitungszeiten und Aushärtegeschwindigkeiten mit N-Furfuryl-1,2-ethandiamin im Vergleich zu N-Benzyl-1,2-ethandiamin nur unwesentlich länger bzw. langsamer sind. Die geringe Exothermie ermöglicht die Verwendung in dickschichtig eingesetzten Epoxidharz-Produkten wie Formkörpern, Vergussmassen oder Matrixharzen für Composites, ohne dass aufgrund hoher Hitzeentwicklung Blasen, Verfärbungen oder sonstige Inhomogenitäten auftreten. Bei flächiger Verwendung ermöglicht der Härter bei Umgebungstemperaturen aushärtende Epoxidharz-Beschichtungen mit schönen, glänzenden Oberflächen und nur sehr wenig Neigung zu Blushing-Effekten. Besonders überraschend ist weiterhin, dass N-Furfuryl-1,2-ethandiamin auch in Form eines wenig aufgereinigten, besonders kostengünstig herstellbaren Reaktionsprodukts verwendet werden kann, ohne nennenswerte Einbussen bei der Aushärtung der Epoxidharze.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist Verwendung eines Härters enthaltend mindestens ein Amin der Formel (I) zum Aushärten von Epoxidharzen, 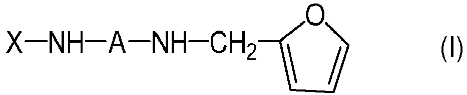 wobei A für einen linearen Alkylenrest mit 2 bis 10 C-Atomen steht und X für H oder Furfuryl steht.

Als "RCI" wird der "Renewable Carbon Index" einer Substanz oder einer Mischung von Substanzen bezeichnet, wobei der RCI für das Verhältnis der Anzahl C-Atome aus biobasierter Quelle zur gesamten Anzahl C-Atome der Substanz oder der Mischung von Substanzen steht.

Als "primäre Aminogruppe" wird eine Aminogruppe bezeichnet, die an einen einzigen organischen Rest gebunden ist und zwei Wasserstoffatome trägt; als "sekundäre Aminogruppe" wird eine Aminogruppe bezeichnet, die an zwei organische Reste, welche auch gemeinsam Teil eines Rings sein können, gebunden ist und ein Wasserstoffatom trägt; und als "tertiäre Aminogruppe" wird eine Aminogruppe bezeichnet, die an drei organische Reste, welche auch zu zweit oder zu dritt Teil eines oder mehrerer Ringe sein können, gebunden ist und kein Wasserstoffatom trägt.

Als "Aminwasserstoff" werden die Wasserstoffatome von primären und sekundären Amingruppen bezeichnet.

Als "Aminwasserstoff-Equivalentgewicht" wird die Masse eines Amins oder einer Amin-haltigen Zusammensetzung, die ein Molequivalent Aminwasserstoff enthält, bezeichnet. Es wird angegeben in der Masseinheit "g/eq".

Als "Epoxid-Equivalentgewicht" wird die Masse einer Epoxidgruppen-haltigen Verbindung oder Zusammensetzung bezeichnet, die ein Mol-Equivalent Epoxidgruppen enthält. Es wird angegeben in der Masseinheit "g/eq".

Mit "Poly" beginnende Substanznamen wie Polyamin oder Polyepoxid bezeichnen Substanzen, die formal zwei oder mehr der in ihrem Namen vorkommenden funktionellen Gruppen pro Molekül enthalten.

Als "Verdünner" wird eine in einem Epoxidharz lösliche und dessen Viskosität senkende Substanz bezeichnet, welche bei der Aushärtung chemisch nicht in das Epoxidharz-Polymer eingebunden wird.

Als "Molekulargewicht" wird die molare Masse (in Gramm pro Mol) eines Moleküls bezeichnet. Als "mittleres Molekulargewicht" wird das Zahlenmittel Mₙ einer polydispersen Mischung von oligomeren oder polymeren Molekülen bezeichnet, welches üblicherweise mittels Gelpermeationschromatographie (GPC) gegen Polystyrol als Standard bestimmt wird.

Als "Topfzeit" wird die maximale Zeitspanne ab dem Mischen der Komponenten und der Applikation einer Epoxidharz-Zusammensetzung bezeichnet, in der die vermischte Zusammensetzung in einem ausreichend fliessfähigen Zustand ist und die Substratoberflächen gut benetzen kann.

Als "Gelierzeit" wird die Zeitspanne ab dem Mischen der Komponenten einer Epoxidharz-Zusammensetzung bis zu deren Gelieren bezeichnet.

Als "Raumtemperatur" wird eine Temperatur von 23 °C bezeichnet.

Alle im Dokument erwähnten Industriestandards und Normen beziehen sich auf die zum Zeitpunkt der Einreichung der Erstanmeldung gültigen Fassungen, sofern nicht anders angegeben.

Gewichtsprozente (Gewichts-%) bezeichnen Massenanteile eines Bestandteils einer Zusammensetzung bezogen auf die gesamte Zusammensetzung, falls nichts anderes angegeben ist. Die Begriffe "Masse" und "Gewicht" werden im vorliegenden Dokument synonym benutzt.

Bevorzugt steht A für 1,2-Ethylen, 1,3-Propylen, 1,4-Butylen, 1,5-Pentylen, 1,6-Hexylen, 1,7-Heptylen, 1,8-Octylen, 1,9-Nonylen oder 1,10-Decylen.

Besonders bevorzugt ist A ausgewählt aus der Gruppe bestehend aus 1,2-Ethylen, 1,3-Propylen, 1,4-Butylen, 1,5-Pentylen und 1,6-Hexylen. Diese Amine sind besonders einfach und mit hohem RCI erhältlich und besonders gut verträglich mit Epoxidharzen.

Am meisten bevorzugt steht A für 1,2-Ethylen. Ein solches Amin der Formel (I) ermöglicht Epoxidharz-Zusammensetzung mit besonders schneller und störungsfreier Aushärtung und verfügt über einen besonders hohen RCI, auch für den Fall, dass die C-Atome aus dem Rest A nicht aus einer biobasierten Quelle stammen. Bevorzugt steht X für H. Ein solches Amin der Formel (I) verdünnt das Epoxidharz besonders gut und ermöglicht eine besonders schnelle Aushärtung und besonders hohe Endhärten.

Bevorzugt ist das Amin der Formel (I) ausgewählt aus der Gruppe bestehend aus N-Furfuryl-1,2-ethandiamin, N,N'-Difurfuryl-1,2-ethandiamin, N-Furfuryl-1,3-propandiamin, N,N'-Difurfuryl-1,3-propandiamin, N-Furfuryl-1,4-butandiamin, N,N'-Difurfuryl-1,4-butandiamin, N-Furfuryl-1,5-pentandiamin, N,N'-Difurfuryl-1,5-pentandiamin, N-Furfuryl-1,6-hexandiamin und N,N'-Difurfuryl-1,6-hexandiamin.

Davon bevorzugt ist N-Furfuryl-1,2-ethandiamin oder N,N'-Difurfuryl-1,2-ethandiamin. Besonders bevorzugt ist N-Furfuryl-1,2-ethandiamin.

In einer bevorzugten Ausführungsform der Erfindung wird das Amin der Formel (I) als Mischung aus Amin der Formel (I) mit X = H und Amin der Formel (I) mit X = Furfuryl in einem Gewichtsverhältnis im Bereich von 50/50 bis 98/2, insbesondere 60/40 bis 95/5, eingesetzt. Eine solche Mischung ist besonders kostengünstig herstellbar und ermöglicht eine schnelle und störungsfreie Aushärtung des Epoxidharzes.

Das Amin der Formel (I) wird bevorzugt hergestellt durch reduktive Alkylierung von mindestens einem Amin der Formel H₂N-A-NH₂ mit Furfural und Wasserstoff.

Bevorzugt basiert Furfural auf nachwachsenden Rohstoffen und weist einen RCI von 1 auf. Dies ermöglicht Amine der Formel (I) mit einem hohen RCI.

Im Handel erhältliches Furfural stammt typischerweise aus einer biobasierten Quelle. Grosstechnisch wird Furfural beispielsweise gewonnen aus Hemicellulose von pflanzlichen Materialien, insbesondere durch Einwirkung von Schwefelsäure auf die darin enthaltenen C5-Zucker in einer Dehydratisierung, oder bei der Zellstoffgewinnung nach dem Magnesiumbisulfitverfahren, wo freigesetztes Furfural aus der Kochlauge extrahiert werden kann.

Als Amin der Formel H₂N-A-NH₂ bevorzugt ist 1,2-Ethandiamin, 1,3-Propandiamin, 1,4-Butandiamin, 1,5-Pentandiamin oder 1,6-Hexandiamin, insbesondere 1,2-Ethandiamin.

In einer bevorzugten Ausführungsform der Erfindung stammen auch die Kohlenstoffatome des Amins der Formel H₂N-A-NH₂ aus einer nachwachsenden Quelle. Dies ermöglicht ganz besonders nachhaltige Amine der Formel (I), insbesondere mit einem RCI von 1.

Bevorzugt weist das Amin der Formel (I) einen RCI von mindestens 0.45, bevorzugt mindestens 0.6, insbesondere mindestens 0.7, am meisten bevorzugt von 1, auf.

Die reduktive Alkylierung wird bevorzugt in Anwesenheit eines geeigneten Katalysators durchgeführt. Als Katalysator bevorzugt sind Palladium auf Kohle (Pd/C), Platin auf Kohle (Pt/C), Adams-Katalysator oder Raney-Nickel, insbesondere Palladium auf Kohle oder Raney-Nickel.

Die reduktive Alkylierung wird bevorzugt in einer Druckapparatur bei einem Wasserstoff-Druck von 5 bis 120 bar, insbesondere 10 bis 100 bar, durchgeführt. Dies kann in einem Batch-Prozess oder bevorzugt in einem kontinuierlichen Prozess erfolgen.

Die reduktive Alkylierung wird bevorzugt bei einer Temperatur im Bereich von 40 bis 120°C, insbesondere 60 bis 100°C, durchgeführt.

Je nach Stöchiometrie zwischen dem Amin der Formel H₂N-A-NH₂ und Furfural enthält das erhaltene Reaktionsgemisch unterschiedliche Anteile an einfach alkyliertem Amin der Formel H₂N-A-NH₂, also Amin der Formel (I) mit X = H, und zweifach alkyliertem Amin der Formel H₂N-A-NH₂, also Amin der Formel (I) mit X = Furfuryl.

Für den Fall, dass ein Amin der Formel (I) mit X = Furfuryl hergestellt werden soll, liegt das Molverhältnis des Amins der Formel H₂N-A-NH₂ zu Furfural bevorzugt im Bereich von 0.4 bis 0.7, insbesondere bei 0.5. Ein so erhaltenes Reaktionsgemisch enthält einen besonders hohen Gehalt an Amin der Formel (I) mit X = Furfuryl.

Für den Fall, dass ein Amin der Formel (I) mit X = H hergestellt werden soll, liegt das Molverhältnis des Amins der Formel H₂N-A-NH₂ zu Furfural bevorzugt im Bereich von 1 bis 10, insbesondere 1 bis 5. Ein so erhaltenes Reaktionsgemisch enthält einen hohen Gehalt an Amin der Formel (I) mit X = H.

Überschüssiges Amin der Formel H₂N-A-NH₂ wird nach der Umsetzung bevorzugt aus der Reaktionsmischung entfernt, insbesondere mittels Destillation zusammen mit dem freigesetzten Wasser.

Die Reaktionsmischung kann weiter gereinigt werden, insbesondere durch Destillation bzw. Fraktionierung. Dabei kann das Amin der Formel (I) von den Nebenprodukten befreit und/oder das Amin der Formel (I) mit X = H vom Amin der Formel (I) mit X = Furfuryl getrennt werden.

Bevorzugt wird das Amin der Formel (I) in Form eines Reaktionsprodukts erhalten aus der reduktiven Alkylierung von mindestens einem Amin der Formel H₂N-A-NH₂ mit Furfural und Wasserstoff und nachfolgender Entfernung von unreagiertem Amin der Formel H₂N-A-NH₂ eingesetzt.

Das Reaktionsprodukt wird dabei bevorzugt nicht weiter aufgereinigt, insbesondere wird auf eine Destillation bzw. Fraktionierung der Amine der Formel (I) verzichtet.

Ein solches Reaktionsprodukt ist besonders kostengünstig herstellbar. Es enthält einen geringen Gehalt an Amin der Formel H₂N-A-NH₂, bevorzugt weniger als 2 Gewichts-%, besonders bevorzugt weniger als 1 Gewichts-%, insbesondere weniger als 0.5 Gewichts-%, Amin der Formel H₂N-A-NH₂ bezogen auf das gesamte Reaktionsprodukt.

Das Reaktionsprodukt kann Nebenprodukte aus der reduktiven Alkylierung enthalten, insbesondere Amine mit doppelt oder dreifach alkylierten Stickstoffatomen sowie Amine mit hydriertem Furanring. Bevorzugt sind diese Anteile gering.

Bevorzugt weist das Reaktionsprodukt einen Gehalt an Aminen mit doppelt oder dreifach alkylierten Stickstoffatomen, insbesondere der Formeln 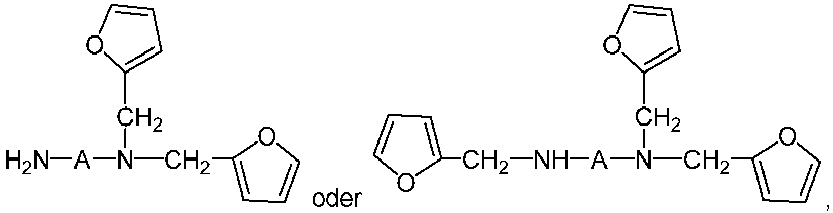 von insgesamt weniger als 10 Gewichts-%, besonders bevorzugt weniger als 5 Gewichts-%, insbesondere weniger als 2 Gewichts-%, bezogen auf das gesamte Reaktionsprodukt, auf.

Bevorzugt weist das Reaktionsprodukt einen Gehalt an Aminen mit hydriertem Furanring, insbesondere der Formeln 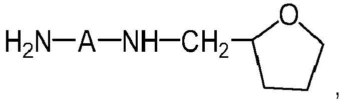 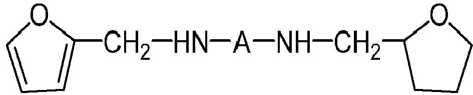 oder 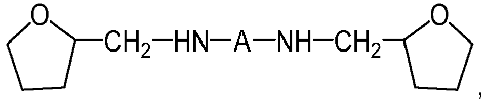 von insgesamt weniger als 20 Gewichts-%, insbesondere weniger als 15 Gewichts-%, bezogen auf das gesamte Reaktionsprodukt, auf.

In einer besonders bevorzugten Ausführungsform der Erfindung wird das Amin der Formel (I) in Form eines Reaktionsprodukts erhalten aus der reduktiven Alkylierung von mindestens einem Amin der Formel H₂N-A-NH₂ mit Furfural und Wasserstoff und nachfolgender Entfernung von unreagiertem Amin der Formel H₂N-A-NH₂ verwendet, wobei das Molverhältnis des Amins der Formel H₂N-A-NH₂ zu Furfural im Bereich von 1 bis 2, bevorzugt 1 bis 1.5, liegt. Bevorzugt steht A dabei für 1,2-Ethylen.

Der Gehalt an Amin der Formel H₂N-A-NH₂ in diesem Reaktionsprodukt beträgt bevorzugt höchstens 1 Gewichts-%, besonders bevorzugt höchstens 0.5 Gewichts-%, insbesondere höchstens 0.2 Gewichts-%, bezogen auf das gesamte Reaktionsprodukt.

Ein solches Reaktionsprodukt enthält einen überraschend hohen Gehalt an Amin der Formel (I) mit X = H und überraschend wenig Amin der Formel (I) mit X = Furfuryl und weist gegenüber dem Epoxidharz eine überraschend hohe Reaktivität auf, die der von weitgehend reinem Amin der Formel (I) mit X = H kaum nachsteht. Dies konnte aus dem Stand der Technik nicht erwartet werden. Eine entsprechende Umsetzung von 1,2-Ethandiamin mit Benzaldehyd anstelle von Furfural weist bei einer entsprechenden Stöchiometrie einen massiv höheren Gehalt an N, N'-dialkyliertem 1,2-Ethandiamin auf.

Bevorzugt liegt das Gewichtsverhältnis zwischen dem Amin der Formel (I) mit X = H und dem Amin der Formel (I) mit X = Furfuryl im Reaktionsprodukt im Bereich von 50/50 bis 98/2, bevorzugt 60/40 bis 95/5 bezogen auf das Reaktionsprodukt.

Ein weiterer Gegenstand der Erfindung ist daher das Reaktionsprodukt, erhalten aus der reduktiven Alkylierung eines Amins der Formel H₂N-A-NH₂ mit Furfural und Wasserstoff in einem Molverhältnis des Amins der Formel H₂N-A-NH₂ zu Furfural im Bereich von 1 bis 2, bevorzugt 1 bis 1.5, und nachfolgender Entfernung von Amin der Formel H₂N-A-NH₂ bis zu einem Gehalt von höchstens 1 Gewichts-%, bevorzugt höchstens 0.5 Gewichts-%, insbesondere höchstens 0.2 Gewichts-%, bezogen auf das Reaktionsprodukt, wobei A für einen linearen Alkylenrest mit 2 bis 10 C-Atomen, insbesondere für 1,2-Ethylen, steht.

Bevorzugt steht A dabei für 1,2-Ethylen und das Reaktionsprodukt enthält
50 bis 80 Gewichts-% N-Furfuryl-1,2-ethandiamin,
5 bis 50 Gewichts-%, insbesondere 5 bis 40 Gewichts-%, N,N'-Difurfuryl-1,2-ethandiamin,
0 bis 20 Gewichts-%, insbesondere 2 bis 15 Gewichts-%, N-Tetrahydrofurfuryl-1,2-ethandiamin,
weniger als 1 Gewichts-%, bevorzugt weniger als 0.5 Gewichts-%, insbesondere weniger als 0.2 Gewichts-%, 1,2-Ethandiamin
und gegebenenfalls weitere Bestandteile, insbesondere weitere Nebenprodukte aus der reduktiven Alkylierung, bezogen auf das Reaktionsprodukt.

Ein solches Reaktionsprodukt ist einfach und kostengünstig herstellbar und ohne weitere Aufreinigung bestens geeignet als Bestandteil eines Härters zum Aushärten von Epoxidharzen, wobei es gegenüber dem Epoxidharz eine hohe Reaktivität aufweist, die der von weitgehend reinem N-Furfuryl-1,2-ethandiamin kaum nachsteht.

Bevorzugt wird ein Amin der Formel (I) mit X = H eingesetzt als Mischung mit einem Amin der Formel 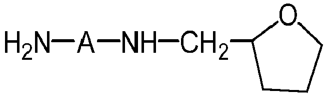 in einem Gewichtsverhältnis zwischen dem Amin der Formel (I) mit X = H und dem Amin der Formel 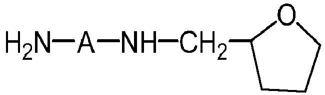 im Bereich von 70/30 bis 99/1, bevorzugt 80/20 bis 98/2, wobei A die bereits genannten Bedeutungen aufweist.

Ein solches Amingemisch ist einfach herstellbar und ermöglicht eine überraschend schnelle und störungsfreie Aushärtung des Epoxidharzes.

Ein weiterer Gegenstand der Erfindung ist somit ein Amingemisch enthaltend mindestens ein Amin der Formel 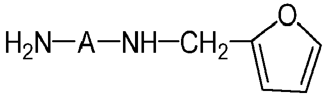 und mindestens ein Amin der Formel 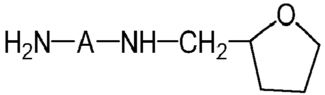 in einem Gewichtsverhältnis im Bereich von 70/30 bis 99/1, bevorzugt 80/20 bis 98/2, wobei A für einen linearen Alkylenrest mit 2 bis 10 C-Atomen, insbesondere für 1,2-Ethylen, steht.

In einer bevorzugten Ausführungsform der Erfindung wird das Amin der Formel (I) teilweise oder vollständig in Form eines aminfunktionellen Addukts mit mindestens einem Epoxidharz oder Monoepoxid in einem stöchiometrischen Verhältnis von mindestens 1 mol Amin der Formel (I) auf 1 Mol-Equivalent Epoxidgruppen verwendet.

Ein solches Addukt liegt als Mischung aus adduktierten Molekülen mit mindestens zwei, typischerweise mit 3 oder 4, vom Amin der Formel (I) abgeleiteten Aminwasserstoffen und freiem, nicht adduktiertem Amin der Formel (I) vor. Es ermöglicht eine besonders schnelle Aushärtung bei moderater Viskosität, insbesondere auch in der Kälte bei 8 °C.

Das Epoxidharz hat bevorzugt ein mittleres Epoxid-Equivalentgewicht im Bereich von 150 bis 500 g/eq, bevorzugt 156 bis 250 g/eq.

Bevorzugt sind aromatische Epoxidharze mit einer mittleren Funktionalität im Bereich von 2 bis 4 ist, insbesondere ein Bisphenol A, F oder A/F Diglycidylether oder ein Novolak Epoxidaharz. Diese Addukte ermöglichen eine besonders schnelle Aushärtung und hohe Glasübergangstemperaturen.

Weiterhin bevorzugt sind Epoxidharze mit Polyoxypropylen- und/oder Polyoxyethylen-Einheiten. Dies sind insbesondere Diglycidylether von Polypropylenglykolen oder Umsetzungsprodukte von Bisphenol A-, F- oder A/F-Diglycidylethern mit Polypropylenglykolen oder Polyethylenglykolen. Solche Addukte sind besonders geeignet als Bestandteil von wasserbasierten Härtern für Epoxidharze. Besonders bevorzugt sind aromatische Diepoxide, insbesondere ein Bisphenol A, F oder A/F Diglycidylether.

Ganz besonders bevorzugt ist ein Bisphenol A Diglycidylether mit einem RCI von 0.28 aus der Umsetzung von Bisphenol-A mit biobasiertem Epichlorhydrin. Damit werden besonders nachhaltige Addukte erhalten.

Bevorzugt erfolgt die Adduktierung ist einem stöchiometrischen Verhältnis im Bereich von 1 bis 10, bevorzugt 1.2 bis 5, insbesondere 1.4 bis 3, mol Amin der Formel (I) pro Molequivalent Epoxidgruppen.

Der Härter enthält bevorzugt mindestens einen weiteren Bestandteil ausgewählt aus weiteren Aminen, welche nicht der Formel (I) entsprechen, Beschleunigern und Verdünnern, insbesondere mindestens ein weiteres Amin, welches nicht der Formel (I) entspricht.

Bevorzugt enthält der Härter mindstens ein weiteres Amin, welches nicht der Formel (I) entspricht und welches kein Nebenprodukt aus der Herstellung des Amins der Formel (I) ist.

Als weiteres Amine, welches nicht der Formel (I) entspricht, bevorzugt sind Amine mit aliphatischen Aminogruppen und mindestens drei Aminwasserstoffen, insbesondere N-Benzyl-1,2-ethandiamin, N-Benzyl-1,2-propandiamin, N-Benzyl-1,3-bis(aminomethyl)benzol, N-(2-Ethylhexyl)-1,3-bis(aminomethyl)benzol, 2,2-Dimethyl-1,3-propandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neo-diamin), 1,6-Hexandiamin, 2,5-Dimethyl-1,6-hexandiamin, 2,2(4),4-Trimethyl-1,6-hexandiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- oder 1,4-Diaminocyclohexan, 1,3-Bis(aminomethyl)cyclohexan, 1,4-Bis(aminomethyl)cyclohexan, Bis(4-aminocyclohexyl)methan, Bis(4-amino-3-methylcyclohexyl)methan, Bis(4-amino-3-ethylcyclohexyl)methan, Bis(4-amino-3,5-dimethylcyclohexyl)-methan, Bis(4-amino-3-ethyl-5-methylcyclohexyl)methan, 1-Amino-3-aminomethyl-3,5,5-trimethylcyclohexan (IPDA), 2(4)-Methyl-1,3-diaminocyclohexan, 2,5(2,6)-Bis(aminomethyl)bicyclo[2.2.1]heptan (NBDA), 3(4),8(9)-Bis(aminomethyl)tricyclo-[5.2.1.0^{2,6}]decan, 1,4-Diamino-2,2,6-trimethylcyclohexan (TMCDA), 1,8-Menthandiamin, 3,9-Bis(3-aminopropyl)-2,4,8,10-tetraoxaspiro[5.5]undecan, 1,3-Bis(aminomethyl)benzol (MXDA), 1,4-Bis(aminomethyl)benzol, Bis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin oder höhere Oligomere dieser Diamine, Bis(3-aminopropyl)-polytetrahydrofurane oder andere Polytetrahydrofurandiamine, Polyoxyalkylendioder -triamine, insbesondere Polyoxypropylendiamine oder Polyoxypropylentriamine wie Jeffamine^{®} D-230, Jeffamine^{®} D-400 oder Jeffamine^{®} T-403 (alle von Huntsman), Furan-basierte Amine wie 2,5-Bis(aminomethyl)furan, 2,5-Bis(aminomethyl)tetrahydrofuran, Bis(5-aminomethylfuran-2-yl)methan, Bis(5-aminomethyl-tetrahydrofuran-2-yl)methan, 2,2-Bis(5-aminomethylfuran-2-yl)propan oder 2,2-Bis(5-aminomethyltetrahydrofuran-2-yl)propan, oder Diethylentriamin (DETA), Triethylentetramin (TETA), Tetraethylenpentamin (TEPA), Pentaethylenhexamin (PEHA), Dipropylentriamin (DPTA), N-(2-Aminoethyl)-1,3-propandiamin (N3-Amin), N,N'-Bis(3-aminopropyl)ethylendiamin (N4-Amin), N,N'-Bis(3-aminopropyl)-1,4-diaminobutan, N5-(3-Aminopropyl)-2-methyl-1,5-pentandiamin, N3-(3-Amino-pentyl)-1,3-pentandiamin, N5-(3-Amino-1-ethylpropyl)-2-methyl-1,5-pentandiamin, N,N'-Bis(3-amino-1-ethylpropyl)-2-methyl-1,5-pentandiamin, 3-(2-Aminoethyl)aminopropylamin, Bis(hexamethylen)triamin (BHMT), N-Aminoethylpiperazin, 3-Dimethylaminopropylamin (DMAPA), 3-(3-(Dimethylamino)propylamino)propylamin (DMAPAPA), aminfunktionelle Addukte der genannten Amine mit Epoxiden, Phenalkamine, welche Umsetzungsprodukte von Cardanol mit Aldehyden, insbesondere Formaldehyd, und Polyaminen darstellen, oder eine Mischung aus zwei oder mehr dieser Amine.

Bevorzugt enthält der Härter mindestens ein Amin ausgewählt aus der Gruppe bestehend aus N-Benzyl-1,2-ethandiamin, N,N'-Dibenzyl-1,2-ethandiamin, MPMD, TMD, 1,2-Diaminocyclohexan, 1,3-Bis(aminomethyl)cyclohexan, 1,4-Bis(amino-methyl)cyclohexan, Bis(4-aminocyclohexyl)methan, IPDA, 2(4)-Methyl-1,3-diaminocyclohexan, MXDA, DETA, TETA, TEPA, N3-Amin, N4-Amin, DPTA, BHMT, Polyoxypropylendiaminen mit einem mittlerem Molekulargewicht Mₙ im Bereich von 200 bis 500 g/mol, Polyoxypropylentriaminen mit einem mittlerem Molekulargewicht Mₙ im Bereich von 300 bis 500 g/mol, 2,5-Bis(aminomethyl)-furan, 2,5-Bis(aminomethyl)tetrahydrofuran, Bis(5-aminomethylfuran-2-yl)methan, Bis(5-aminomethyltetrahydrofuran-2-yl)methan, 2,2-Bis(5-aminomethylfuran-2-yl)propan, 2,2-Bis(5-aminomethyltetrahydrofuran-2-yl)propan und Phenalkaminen.

Davon bevorzugt ist 1,3-Bis(aminomethyl)cyclohexan oder 1,4-Bis(aminomethyl)-cyclohexan, insbesondere 1,3-Bis(aminomethyl)cyclohexan. Damit wird eine besonders schnelle Aushärtung ermöglicht.

Davon weiterhin bevorzugt ist IPDA. Damit werden besonders hohe Glasübergangstemperaturen erreicht, was eine besonders gute Robustheit gegenüber hohen Gebrauchstemperaturen ermöglicht. Besonders bevorzugt wird IPDA mit einem hohen RCI aus biobasiertem Aceton eingesetzt, was besonders nachhaltige Härter ermöglicht.

Davon weiterhin bevorzugt ist MXDA. Damit werden hohe Aushärtegeschwindigkeiten und besonders hohe Festigkeiten erreicht.

Davon weiterhin bevorzugt ist N-Benzyl-1,2-ethandiamin. Ein solcher Härter ermöglicht besonders niedrigviskose Epoxidharz-Produkte mit besonders schönen Oberflächen.

Davon weiterhin bevorzugt ist 2,5-Bis(aminomethyl)furan, 2,5-Bis(aminomethyl)-tetrahydrofuran, Bis(5-aminomethylfuran-2-yl)methan, Bis(5-aminomethyltetra-hydrofuran-2-yl)methan, 2,2-Bis(5-aminomethylfuran-2-yl)propan oder 2,2-Bis(5-aminomethyltetrahydrofuran-2-yl)propan, insbesondere 2,5-Bis(aminomethyl)-furan. Damit werden besonders nachhaltige Härter ermöglicht.

Der Härter kann insbesondere mehr als ein weiteres Amin, welches nicht der Formel (I) entspricht, enthalten.

Besonders bevorzugt enthält der Härter als weiteres Amin, welches nicht der Formel (I) entspricht, mindestens ein Amin mit einem RCI von 1, insbesondere ausgewählt aus 2,5-Bis(aminomethyl)furan, 2,5-Bis(aminomethyl)tetrahydrofuran, Bis(5-aminomethylfuran-2-yl)methan, Bis(5-aminomethyltetrahydrofuran-2-yl)-methan, 2,2-Bis(5-aminomethylfuran-2-yl)propan und 2,2-Bis(5-aminomethyltetrahydrofuran-2-yl)propan.

Bevorzugt enthält der Härter eine solche Menge an weiteren Aminen, welche nicht der Formel (I) entsprechen, dass 5 bis 95 %, bevorzugt 10 bis 80 %, insbesondere 15 bis 60 %, aller vorhandenen Aminwasserstoffe von Aminen der Formel (I) stammen. Für den Fall, dass das Amin der Formel (I) in mit einem Epoxidharz adduktierter Form vorliegt, werden die Aminwasserstoffe solcher Addukte ebenfalls als Aminwasserstoffe des Amins der Formel (I) gezählt.

Geeignete Beschleuniger sind insbesondere Säuren oder zu Säuren hydrolysierbare Verbindungen, insbesondere organische Carbonsäuren wie Essigsäure, Benzoesäure, Salicylsäure, 2-Nitrobenzoesäure, Milchsäure, organische Sulfonsäuren wie Methansulfonsäure, p-Toluolsulfonsäure oder 4-Dodecylbenzolsulfonsäure, Sulfonsäureester, andere organische oder anorganische Säuren wie insbesondere Phosphorsäure, oder Mischungen der vorgenannten Säuren und Säureester; Nitrate wie insbesondere Calciumnitrat; tertiäre Amine wie insbesondere 1,4-Diazabicyclo[2.2.2]octan, Benzyldimethylamin, α-Methylbenzyldimethylamin, Triethanolamin, Dimethylaminopropylamin, Imidazole wie insbesondere N-Methylimidazol, N-Vinylimidazol oder 1,2-Dimethylimidazol, Salze solcher tertiärer Amine, quaternäre Ammoniumsalze, wie insbesondere Benzyltrimethylammoniumchlorid, Amidine wie insbesondere 1,8-Diazabicyclo[5.4.0]-undec-7-en, Guanidine wie insbesondere 1,1,3,3-Tetramethylguanidin, Phenole, insbesondere Bisphenole, Phenol-Harze oder Mannich-Basen wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin, Phosphite wie insbesondere Di- oder Triphenylphosphite, oder Mercaptogruppen aufweisende Verbindungen.

Bevorzugt sind Säuren, Nitrate, tertiäre Amine oder Mannich-Basen, insbesondere Salicylsäure, Calciumnitrat oder 2,4,6-Tris(dimethylaminomethyl)phenol, oder eine Kombination dieser Beschleuniger.

Geeignete Verdünner sind insbesondere n-Propanol, Isopropanol, n-Butanol, Isobutanol, tert.Butanol, 1-Pentanol, 2-Pentanol, 3-Pentanol, 2-Methyl-1-butanol, 3-Methyl-1-butanol, 3-Methyl-2-butanol n-Hexanol, 2-Ethylhexanol, Xylol, 2-Methoxyethanol, Dimethoxyethanol, 2-Ethoxyethanol, 2-Propoxyethanol, 2-Isopropoxyethanol, 2-Butoxyethanol, 2-Phenoxyethanol, 2-Benzyloxyethanol, Benzylalkohol, Ethylenglykol, Ethylenglykoldimethylether, Ethylenglykoldiethylether, Ethylenglykoldibutylether, Ethylenglykoldiphenylether, Diethylenglykol, Diethylenglykolmonomethylether, Diethylenglykolmonoethylether, Diethylenglykolmono-n-butylether, Diethylenglykoldimethylether, Diethylenglykoldiethylether, Diethylenglykoldi-n-butylylether, Propylenglykolbutylether, Propylenglykolphenylether, Dipropylenglykol, Dipropylenglykolmonomethylether, Dipropylenglykoldimethylether, Dipropylenglykoldi-n-butylether, 2,2,4-Trimethyl-1,3-pentandiolmonoisobutyrat, Diphenylmethan, Diisopropylnaphthalin, Erdölfraktionen wie zum Beispiel Solvesso^{®}-Typen (von Exxon), Alkylphenole wie tert.Butylphenol, Nonylphenol, Dodecylphenol, Cardanol, styrolisiertes Phenol, Bisphenole, aromatische Kohlenwasserstoffharze, insbesondere Phenolgruppen-haltige Typen, alkoxyliertes Phenol, insbesondere ethoxyliertes oder propoxyliertes Phenol, insbesondere 2-Phenoxyethanol, Adipate, Sebacate, Phthalate, Benzoate, organische Phosphor- oder Sulfonsäureester oder Sulfonamide.

Davon bevorzugt sind Verdünner mit einem Siedepunkt von mehr als 200 °C, insbesondere Benzylalkohol, styrolisiertem Phenol, ethoxyliertes Phenol, phenolgruppenhaltige aromatische Kohlenwasserstoffharze wie insbesondere die Novares^{®}-Typen LS 500, LX 200, LA 300 oder LA 700 (von Rütgers), Diisopropylnaphthalin oder Cardanol, insbesondere Benzylalkohol. Phenolgruppen-haltige Verdünner zeigen auch eine Wirkung als Beschleuniger. Davon bevorzugt sind weiterhin aromatische Verdünner mit einer besonders hohen verdünnenden Wirkung, insbesondere Xylol.

Davon besonders bevorzugt sind Verdünner mit einem RCI von 1, insbesondere Cardanol. Diese ermöglichen einen besonders nachhaltigen Härter.

Bevorzugt enthält der Härter nur einen geringen Gehalt an Verdünnern, insbesondere 0 bis 50 Gewichts-%, bevorzugt 0 bis 30 Gewichts-%, Verdünner bezogen auf den gesamten Härter.

Der Härter enthält bevorzugt 1 bis 99 Gewichts-%, mehr bevorzugt 5 bis 90 Gewichts-%, mehr bevorzugt 10 bis 80 Gewichts-%, besonders bevorzugt 15 bis 70 Gewichts-%, Amine der Formel (I) bezogen auf den gesamten Härter.

Der Härter kann wasserbasiert sein und Wasser im Bereich von 15 bis 90 Gewichts-%, bevorzugt 20 bis 80 Gewichts-%, enthalten.

Der Härter ist bevorzugt nicht wasserbasiert. Er enthält bevorzugt weniger als 15 Gewichts-%, insbesondere weniger als 10 Gewichts-%, Wasser, bezogen auf den gesamten Härter. Ein solcher Härter ist besonders geeignet für nicht-wässrige Epoxidharz-Produkte.

Der Härter kann weitere Bestandteile enthalten, insbesondere:
- weitere Addukte, insbesondere Addukte von MPMD oder 1,2-Ethandiamin oder 1,2-Propandiamin mit Kresylglycidylether oder aromatischen Epoxidharzen, bei welchen nicht umgesetztes MPMD, 1,2-Ethandiamin oder 1,2-Propandiamin nach der Umsetzung destillativ entfernt wurde,
- Monoamine wie insbesondere Benzylamin oder Furfurylamin,
- Polyamidoamine, insbesondere Umsetzungsprodukte aus einer ein- oder mehrwertigen Carbonsäure oder deren Ester oder Anhydrid, insbesondere einer Dimerfettsäure, mit einem im stöchiometrischen Überschuss eingesetzten Polyamin, insbesondere DETA oder TETA,
- Mannich-Basen,
- aromatische Polyamine wie insbesondere 4,4'-, 2,4' und/oder 2,2'-Diaminodiphenylmethan, 2,4(6)-Toluoldiamin, 3,5-Dimethylthio-2,4(6)-toluoldiamin oder 3,5-Diethyl-2,4(6)-toluylendiamin,
- Mercaptogruppen aufweisende Verbindungen, insbesondere flüssige Mercaptan-terminierte Polysulfidpolymere, Mercaptan-terminierte Polyoxyalkylenether, Mercaptan-terminierte Polyoxyalkylen-Derivate, Polyester von Thiocarbonsäuren, 2,4,6-Trimercapto-1,3,5-triazin, Triethylenglykoldimercaptan oder Ethandithiol,
- oberflächenaktive Additive, insbesondere Entschäumer, Entlüfter, Netzmittel, Dispergiermittel oder Verlaufsmittel, oder
- Stabilisatoren, insbesondere Stabilisatoren gegen Oxidation, Wärme, Licht oder UV-Strahlung.

Ein weiterer Gegenstand der Erfindung ist eine Epoxidharz-Zusammensetzung umfassend
- eine Harz-Komponente umfassend mindestens ein Epoxidharz und
- eine Härter-Komponente umfassend den Härter enthaltend mindestens ein Amin der Formel (I), wie vorgängig beschrieben.

Ein geeignetes Epoxidharz wird auf bekannte Art und Weise erhalten, insbesondere aus der Reaktion von Epichlorhydrin mit Polyolen, Polyphenolen oder Aminen.

Geeignete Epoxidharze sind insbesondere aromatische Epoxidharze, insbesondere die Glycidylether von:
- Bisphenol A, Bisphenol F oder Bisphenol A/F, wobei A für Aceton und F für Formaldehyd steht, welche als Edukte zur Herstellung dieser Bisphenole dienten. Im Fall von Bisphenol F können auch Stellungsisomere vorhanden sein, insbesondere abgeleitet von 2,4'- oder 2,2'-Hydroxyphenylmethan.
- Dihydroxybenzol-Derivaten wie Resorcin, Hydrochinon oder Brenzcatechin;
- weiteren Bisphenolen oder Polyphenolen wie Bis(4-hydroxy-3-methylphenyl)-methan, 2,2-Bis(4-hydroxy-3-methylphenyl)propan (Bisphenol-C), Bis(3,5-dimethyl-4-hydroxyphenyl)methan, 2,2-Bis(3,5-dimethyl-4-hydroxyphenyl)propan, 2,2-Bis(3,5-dibromo-4-hydroxyphenyl)propan, 2,2-Bis(4-hydroxy-3-tert.butylphenyl)propan, 2,2-Bis(4-hydroxyphenyl)butan (Bisphenol-B), 3,3-Bis(4-hydroxyphenyl)pentan, 3,4-Bis(4-hydroxyphenyl)hexan, 4,4-Bis(4-hydroxyphenyl)-heptan, 2,4-Bis(4-hydroxyphenyl)-2-methylbutan, 2,4-Bis(3,5-dimethyl-4-hydroxyphenyl)-2-methylbutan, 1,1-Bis(4-hydroxyphenyl)cyclohexan (Bisphenol-Z), 1,1-Bis(4-hydroxyphenyl)-3,3,5-trimethylcyclohexan (Bisphenol-TMC), 1,1-Bis(4-hydroxyphenyl)-1-phenylethan, 1,4-Bis[2-(4-hydroxyphenyl)-2-propyl]-benzol (Bisphenol-P), 1,3-Bis[2-(4-hydroxyphenyl)-2-propyl]benzol (Bisphenol-M), 4,4'-Dihydroxydiphenyl (DOD), 4,4'-Dihydroxybenzophenon, Bis(2-hydroxy-naphth-1-yl)methan, Bis(4-hydroxynaphth-1-yl)methan, 1,5-Dihydroxynaphthalin, Tris(4-hydroxyphenyl)methan, 1,1,2,2-Tetrakis(4-hydroxyphenyl)ethan, Bis(4-hydroxyphenyl)ether oder Bis(4-hydroxyphenyl)sulfon;
- Novolaken, welche insbesondere Kondensationsprodukte von Phenol oder Kresolen mit Formaldehyd bzw. Paraformaldehyd oder Acetaldehyd oder Crotonaldehyd oder Isobutyraldehyd oder 2-Ethylhexanal oder Benzaldehyd oder Furfural sind;
- aromatischen Aminen, wie Anilin, Toluidin, 4-Aminophenol, 4,4'-Methylendiphenyldiamin, 4,4'-Methylendiphenyldi-(N-methyl)amin, 4,4'-[1,4-Phenylenbis(1-methylethyliden)]bisanilin (Bisanilin-P) oder 4,4'-[1,3-Phenylen-bis(1-methylethyliden)]bisanilin (Bisanilin-M).

Weitere geeignete Epoxidharze sind aliphatische oder cycloaliphatische Polyepoxide, insbesondere
- Glycidylether von gesättigten oder ungesättigten, verzweigten oder unverzweigten, zyklischen oder offenkettigen di-, tri- oder tetrafunktionellen C₂- bis C₃₀-Alkoholen, insbesondere Ethylenglykol, Propylenglykol, Butylenglykol, Hexandiol, Octandiol, Polypropylenglykolen, Dimethylolcyclohexan, Neopentylglykol, Dibromoneopentylglykol, Rizinusöl, Trimethylolpropan, Trimethylolethan, Pentaerythrol, Sorbit oder Glycerin, oder alkoxyliertes Glycerin oder alkoxyliertes Trimethylolpropan;
- ein hydriertes Bisphenol-A-, -F- oder -A/F-Flüssigharz, beziehungsweise die Glycidylisierungsprodukte von hydriertem Bisphenol-A, -F oder -A/F;
- ein N-Glycidylderivat von Amiden oder heterocyclischen Stickstoffbasen, wie Triglycidylcyanurat oder Triglycidylisocyanurat, oder Umsetzungsprodukte von Epichlorhydrin mit Hydantoin.

Bevorzugt ist das Epoxidharz ein Flüssigharz oder eine Mischung enthaltend zwei oder mehr Epoxid-Flüssigharze.

Als "Epoxid-Flüssigharz" wird ein technisches Polyepoxid mit einer Glasübergangstemperatur unterhalb von 25 °C bezeichnet.

Gegebenenfalls enthält die Harz-Komponente zusätzlich Anteile von Epoxid-Festharz.

Das Epoxidharz ist insbesondere ein Flüssigharz auf der Basis eines Bisphenols oder Novolaks, insbesondere mit einem mittleren Epoxid-Equivalentgewicht im Bereich von 156 bis 210 g/eq.

Besonders geeignet ist ein Bisphenol-A Diglycidylether und/oder Bisphenol-F-Diglycidylether, wie sie kommerziell beispielsweise von Olin, Huntsman oder Momentive erhältlich sind. Diese Flüssigharze weisen eine für Epoxidharze niedrige Viskosität auf und ermöglichen eine schnelle Aushärtung und hohe Härten. Sie können Anteile von Bisphenol A-Festharz oder Novolak-Epoxidharzen enthalten.

Ganz besonders bevorzugt ist ein Bisphenol-A Diglycidylether mit einem RCI von 0.28 aus der Umsetzung von Bisphenol-A mit biobasiertem Epichlorhydrin. Dies ermöglicht eine besonders nachhaltige Epoxidharz-Zusammensetzung.

Weiterhin besonders geeignet sind Phenol-Formaldehyd Novolak-Glycidylether, insbesondere mit einer mittleren Funktionalität im Bereich von 2.3 bis 4, bevorzugt 2.5 bis 3. Sie können Anteile von weiteren Epoxidharzen enthalten, insbesondere Bisphenol-A Diglycidylether oder Bisphenol-F-Diglycidylether.

Die Harz-Komponente kann einen Reaktivverdünner enthalten.

Als Reaktivverdünner bevorzugt sind Epoxidgruppen-haltige Reaktivverdünner, insbesondere Butandioldiglycidylether, Hexandioldiglycidylether, Trimethylolpropandi- oder triglycidylether, Phenylglycidylether, Kresylglycidylether, Guaiacolglycidylether, 4-Methoxyphenylglycidylether, p-n-Butylphenylglycidylether, p-tert.Butylphenylglycidylether, 4-Nonylphenylglycidylether, 4-Dodecylphenylglycidylether, Cardanolglycidylether, Benzylglycidylether, Allylglycidylether, Butylglycidylether, Hexylglycidylether, 2-Ethylhexylglycidylether, oder Glycidylether von natürlichen Alkoholen wie insbesondere Ca- bis C₁₀- oder C₁₂- bis C₁₄- oder C₁₃-bis C₁₅-Alkylglycidylether.

Bevorzugt enthält die Epoxidharz-Zusammensetzung mindestens einen weiteren Bestandteil ausgewählt aus der Gruppe bestehend aus Verdünnern, Beschleunigern, Füllstoffen, Pigmenten und oberflächenaktiven Additiven.

Als Verdünnern oder Beschleuniger geeignet sind insbesondere die bereits genannten.

Geeignete Füllstoffe sind insbesondere gemahlenes oder gefälltes Calciumcarbonat, welches gegebenenfalls mit Fettsäure, insbesondere Stearaten, beschichtet ist, Baryt (Schwerspat), Talk, Quarzmehl, Quarzsand, Siliciumcarbid, Eisenglimmer, Dolomit, Wollastonit, Kaolin, Mica (Kalium-Aluminium-Silikat), Molekularsiebe, Aluminiumoxid, Zinkoxid, Aluminium-dotiertes Zinkoxid, Aluminiumhydroxid, Magnesiumhydroxid, Kieselsäure, Zement, Gips, Flugasche, Russ, Graphit, Metall-Pulver wie Aluminium, Kupfer, Eisen, Zink, Silber oder Stahl, PVC-Pulver oder Hohlkugeln. Davon bevorzugt sind Calciumcarbonat, Baryt, Quarzmehl, Talk, Aluminiumpulver oder eine Kombination davon.

Geeignete Pigment sind insbesondere Titandioxide, Eisenoxide, Chrom(III)oxide, organische Pigmente, Russ oder Korrosionsschutzpigmente, insbesondere Phosphate, Orthophosphate oder Polyphosphate, welche als Gegenion insbesondere Chrom, Zink, Aluminium, Kalzium, Strontium oder eine Kombination dieser Metalle enthalten. Besonders geeignet sind Titandioxide.

Geeignete oberflächenaktive Additive sind insbesondere Entschäumer, Entlüfter, Netzmittel, Dispergiermittel, Verlaufsmittel und/oder dispergierte Paraffinwachse.

Gegebenenfalls enthält die Epoxidharz-Zusammensetzung weitere Hilfs- und Zusatzstoffe, insbesondere die Folgenden:
- Reaktivverdünner, insbesondere die bereits vorgängig erwähnten, oder epoxidiertes Sojaöl oder Leinöl, Acetoacetatgruppen aufweisende Verbindungen, insbesondere acetoacetylierte Polyole, Butyrolakton, Carbonate, Aldehyde, Isocyanate oder Reaktivgruppen aufweisende Silikone;
- Polymere, insbesondere Polyamide, Polysulfide, Polyvinylformal (PVF), Polyvinylbutyral (PVB), Polyurethane (PUR), Polymere mit Carboxylgruppen, Polyamide, Butadien-Acrylnitril-Copolymere, Styrol-Acrylnitril-Copolymere, Butadien-Styrol-Copolymere, Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere chlorsulfonierte Polyethylene oder Fluor-haltige Polymere oder Sulfonamid-modifizierte Melamine;
- Fasern, insbesondere Glasfasern, Kohlefasern, Metallfasern, Keramikfasern oder Kunststofffasern wie Polyamidfasern oder Polyethylenfasern;
- Nanofüllstoffe, insbesondere Carbon Nanotubes;
- Rheologie-Modifizierer, insbesondere Verdicker oder Antiabsetzmittel;
- Haftverbesserer, insbesondere Organoalkoxysilane;
- flammhemmende Substanzen, insbesondere die bereits genannten Füllstoffe Aluminiumhydroxid oder Magnesiumhydroxid, Antimontrioxid, Antimonpentoxid, Borsäure (B(OH)₃), Zinkborat, Zinkphosphat, Melaminborat, Melamincyanurat, Ammoniumpolyphosphat, Melaminphosphat, Melaminpyrophosphat, polybromierte Diphenyloxide oder Diphenylether, Phosphate wie insbesondere Diphenylkresylphosphat, Resorcinol-bis(diphenylphosphat), Resorcinoldiphosphat-Oligomer, Tetraphenylresorcinoldiphosphit, Ethylendiamindiphosphat, Bisphenol-A-bis(diphenylphosphat), Tris(chloroethyl)phosphat, Tris(chloropropyl)-phosphat, Tris(dichloroisopropyl)phosphat, Tris[3-bromo-2,2-bis(bromomethyl)-propyl]phosphat, Tetrabromo-Bisphenol-A, Bis(2,3-dibromopropylether) von Bisphenol A, bromierte Epoxidharze, Ethylen-bis(tetrabromophthalimid), Ethylen-bis(dibromonorbornandicarboximid), 1,2-Bis(tribromophenoxy)ethan, Tris(2,3-dibromopropyl)isocyanurat, Tribromophenol, Hexabromocyclododecan, Bis(hexachlorocyclopentadieno)cyclooctan oder Chlorparaffine; oder
- Stabilisatoren gegen Oxidation, Wärme, Licht oder UV-Strahlung oder Biozide.

Bevorzugt enthält die Epoxidharz-Zusammensetzung nur einen geringen Gehalt an Verdünnern. Bevorzugt enthält sie weniger als 20 Gewichts-%, besonders bevorzugt weniger als 10 Gewichts-%, insbesondere weniger als 5 Gewichts-%, , am meisten bevorzugt weniger als 1 Gewichts-% Verdünner.

Die Epoxidharz-Zusammensetzung kann Wasser enthalten.

In einer Ausführungsform ist die Epoxidharz-Zusammensetzung wasserbasiert. Dabei ist das Epoxidharz bevorzugt in einer Menge von 50 bis 85 Gewichts-% in Wasser emulgiert und die Härter-Komponente enthält bevorzugt 20 bis 80 Gewichts-% Wasser.

Bevorzugt enthält die Epoxidharz-Zusammensetzung aber nur einen geringen Gehalt an Wasser, bevorzugt weniger als 5 Gewichts-%, insbesondere weniger als 1 Gewichts-%, Wasser. Eine solche, nicht-wasserbasierte Epoxidharz-Zusammensetzung ist besonder vielseitig verwendbar und besonders wasserbeständig.

Bevorzugt ist eine Epoxidharz-Zusammensetzung umfassend
- eine Harz-Komponente enthaltend mindestens ein Epoxidharz und gegebenenfalls weitere Bestandteile wie insbesondere Epoxidgruppen-haltige Reaktivverdünner, Verdünner, Füllstoffe, Pigmente und/oder oberflächenaktive Additive, und
- eine Härter-Komponente enthaltend mindestens ein Amin der Formel (I) und gegebenenfalls weitere Bestandteile wie insbesondere weitere Amine, Beschleuniger und/oder Verdünner.

Die Harz- und die Härter-Komponente der Epoxidharz-Zusammensetzung werden in voneinander getrennten Gebinden gelagert.

Ein geeignetes Gebinde zum Lagern der Harz- oder der Härter-Komponente ist insbesondere ein Fass, ein Hobbock, ein Beutel, ein Eimer, eine Büchse, eine Kartusche oder eine Tube. Die Komponenten sind lagerfähig, das heisst, dass sie vor ihrer Anwendung während mehreren Monaten bis zu einem Jahr und länger aufbewahrt werden können, ohne dass sie sich in ihren jeweiligen Eigenschaften in einem für ihren Gebrauch relevanten Ausmass verändern.

Die Harz- und die Härter-Komponente werden kurz vor oder während der Applikation vermischt. Das Mischungsverhältnis wird bevorzugt so gewählt, dass das molare Verhältnis der gegenüber Epoxidgruppen reaktiven Gruppen zu den Epoxidgruppen im Bereich von 0.5 bis 1.5, insbesondere 0.7 bis 1.2, liegt. In Gewichtsteilen liegt das Mischungsverhältnis zwischen der Harz- und der Härter-Komponente typischerweise im Bereich von 1:2 bis 20:1.

Das Mischen der Komponenten erfolgt kontinuierlich oder batchweise mittels eines geeigneten Verfahrens, wobei darauf zu achten ist, dass zwischen dem Mischen der Komponenten und der Applikation nicht zu viel Zeit vergeht und die Applikation innerhalb der Topfzeit erfolgt. Das Mischen und die Applikation können bei Umgebungstemperatur erfolgen, welche typischerweise im Bereich von etwa 5 bis 40°C, bevorzugt bei etwa 10 bis 35°C, liegt, oder bei erhöhter Temperatur, insbesondere im Bereich von 40 bis 150 °C, bevorzugt 50 bis 120 °C.

Mit dem Mischen der Komponenten beginnt die Aushärtung der Epoxidharz-Zusammensetzung durch chemische Reaktion. Primäre und sekundäre Aminogruppen und gegebenenfalls vorhandene weitere gegenüber Epoxidgruppen reaktive Gruppen reagieren mit den Epoxidgruppen unter deren Ringöffnung. Als Ergebnis hauptsächlich dieser Reaktionen polymerisiert die Zusammensetzung und härtet dadurch aus.

Die Aushärtung erstreckt sich typischerweise über einige Stunden bis Tage. Die Dauer hängt unter anderem von der Temperatur, der Reaktivität der Bestandteile, deren Stöchiometrie und der Gegenwart bzw. Menge von Beschleunigern ab.

Im frisch vermischten Zustand hat die Epoxidharz-Zusammensetzung eine niedrige Viskosität. Bevorzugt liegt die Viskosität 5 Minuten nach dem Mischen der Harz- und der Härter-Komponente bei 20 °C im Bereich von 0.1 bis 20 Pa·s, bevorzugt 0.2 bis 10 Pa·s, besonders bevorzugt 0.3 bis 5 Pa·s, gemessen mittels Kegel-Platten Viskosimeter bei einer Schergeschwindigkeit von 10 s⁻¹.

Die Applikation der Epoxidharz-Zusammensetzung erfolgt auf mindestens ein Substrat und/oder in mindestens eine Giessform.

Als Substrat geeignet sind insbesondere:
- Glas, Glaskeramik, Beton, Mörtel, Zementestrich, Faserzement, Backstein, Ziegel, Gips oder Natursteine wie Granit oder Marmor;
- Reparatur- oder Nivelliermassen auf Basis PCC (Polymer-modifizierter Zementmörtel) oder ECC (Epoxidharz-modifizierter Zementmörtel);
- Metalle oder Legierungen wie Aluminium, Eisen, Stahl, Kupfer, weitere Buntmetalle, inklusive oberflächenveredelte Metalle oder Legierungen wie verzinkte oder verchromte Metalle;
- Asphalt oder Bitumen;
- Leder, Textilien, Papier, Holz, mit Harzen, beispielsweise Phenol-, Melaminoder Epoxidharzen, gebundene Holzwerkstoffe, Harz-Textil-Verbundwerkstoffe oder weitere sogenannte Polymer-Composites;
- Kunststoffe wie Hart- und Weich-PVC, Polycarbonat, Polystyrol, Polyester, Polyamid, PMMA, ABS, SAN, Epoxidharze, Phenolharze, PUR, POM, TPO, PE, PP, EPM oder EPDM, jeweils unbehandelt oder oberflächenbehandelt, beispielsweise mittels Plasma, Corona oder Flammen;
- Faserverstärkte Kunststoffe, wie Kohlefaser-verstärkte Kunststoffe (CFK), Glasfaser-verstärkte Kunststoffe (GFK) und Sheet Moulding Compounds (SMC);
- Isolierschäume, insbesondere aus EPS, XPS, PUR, PIR, Steinwolle, Glaswolle oder geschäumtem Glas (Foamglas);
- beschichtete oder lackierte Substrate, insbesondere lackierte Fliesen, gestrichener Beton, pulverbeschichtete Metalle oder Legierungen oder lackierte Bleche;
- Beschichtungen, Farben oder Lacke, insbesondere beschichtete Böden, welche mit einer weiteren Bodenbelagsschicht überschichtet werden.

Die Substrate können bei Bedarf vor dem Applizieren vorbehandelt werden, insbesondere durch physikalische und/oder chemische Reinigungsverfahren oder das Aufbringen eines Aktivators oder eines Primers.

Die Substrate werden insbesondere beschichtet und/oder verklebt.

Als Giessform geeignet ist eine Vorrichtung, in welche die vermischte, flüssige Epoxidharz-Zusammensetzung gegossen und darin ausgehärtet werden und nach der Aushärtung daraus entformt bzw. entnommen werden kann, wobei die ausgehärtete Zusammensetzung einen Formkörper bildet.

Die Giessform besteht bevorzugt zumindest auf der Oberfläche aus einem Material, von welchem die ausgehärtete Epoxidharz-Zusammensetzung ohne Beschädigung wieder gelöst werden kann, insbesondere aus Metall, Keramik, Kunststoff oder Silikon, welche gegebenenfalls mit einer Antihaft-Beschichtung versehen sind, insbesondere aus Teflon, Silikon oder einem Wachs.

Ein weiterer Gegenstand der Erfindung ist eine ausgehärtete Zusammensetzung erhalten aus der beschriebenen Epoxidharz-Zusammensetzung nach dem Vermischen der Harz- und der Härter-Komponente.

Die Epoxidharz-Zusammensetzung wird bevorzugt verwendet als Beschichtung, Primer, Klebstoff, Dichtstoff, Vergussmasse, Giessharz, Imprägnierharz, oder als Formkörper oder Matrix für Verbundwerkstoffe (Composites) wie insbesondere CFK (enthaltend Carbonfasern) oder GFK (enthaltend Glasfasern) oder Holzverbundwerkstoff.

Aus der Verwendung entsteht ein Artikel enthaltend die ausgehärtete Zusammensetzung aus der beschriebenen Epoxidharz-Zusammensetzung.

Der Artikel ist insbesondere eine Bodenbeschichtung, Wandbeschichtung, Bauteilbeschichtung, Rohrbeschichtung, Dachbeschichtung oder eine Korrosionsschutzbeschichtung, oder ein verklebter Artikel, oder ein Formkörper, insbesondere ein Composit-Material.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

"AHEW" steht für das Aminwasserstoff-Equivalentgewicht.

"EEW" steht für das Epoxid-Equivalentgewicht.

Als "Normklima" ("NK") wird eine Temperatur von 23±1°C und eine relative Luftfeuchtigkeit von 50±5% bezeichnet.

Die verwendeten Chemikalien waren, sofern nicht anders bezeichnet, von Sigma-Aldrich Chemie GmbH.

### Beschreibung der Messmethoden:

Die **Viskosität** wurde auf einem thermostatisierten Kegel-Platten-Viskosimeter Rheotec RC30 (Kegeldurchmesser 50 mm, Kegelwinkel 1°, Kegelspitze-PlattenAbstand 0.05 mm, Scherrate 10 s⁻¹) gemessen. Viskositäten von weniger als 100 mPa·s wurden mit einer Scherrate von 100 s⁻¹ gemessen.

Die **Aminzahl** wurde durch Titration bestimmt (mit 0.1N HClO₄ in Essigsäure gegen Kristallviolett).

**Gaschromatogramme** (GC) wurden gemessen im Temperaturbereich von 60 bis 320 °C mit einer Aufheizrate von 15 °C/min und 10 min Verweilzeit bei 320 °C. Die Injektortemperatur betrug 250 °C. Es wurde eine Zebron ZB-5 Säule verwendet (L = 30 m, ID = 0.25 mm, dj = 0.5 µm) bei einem Gasfluss von 1.5 ml/Min. Die Detektion erfolgte mittels Flammenionisation (FID).

**Infrarotspektren** (FT-IR) wurden als unverdünnte Filme auf einem mit horizontaler ATR-Messeinheit mit Diamant-Kristall ausgestatteten FT-IR Gerät Nicolet iS5 von Thermo Scientific gemessen. Die Absorptionsbanden sind in Wellenzahlen (cm⁻¹) angegeben.

**¹H-NMR-Spektren** wurden auf einem Spektrometer des Typs Bruker Ascend 400 bei 400.14 MHz gemessen; die chemischen Verschiebungen δ sind angegeben in ppm relativ zu Tetramethylsilan (TMS). Echte und Pseudo-Kopplungsmuster wurden nicht unterschieden.

### Verwendete Substanzen und Abkürzungen:

| | |
|---|---|
| Araldite^{®} GY 250: | Bisphenol A-Diglycidylether, EEW ca. 187 g/eq (von Huntsman) |
| Araldite^{®} DY-E: | Monoglycidylether von C₁₂- bis C₁₄-Alkoholen, EEW ca. 290 g/eq (von Huntsman) |
| D.E.N.^{®} 438: | Phenol-Formaldehyd Novolak-Glycidylether), EEW ca. 179 g/eq, mittlere Funktionalität ca. 3.6 (von Olin) |
| IPDA | 3-Aminomethyl-3,5,5-trimethylcyclohexylamin, AHEW 42.6 g/eq (Vestamin^{®} IPD, von Evonik) |
| MXDA: | 1,3-Bis(aminomethyl)benzol, AHEW 34 g/Eq (von Mitsubishi Gas Chemical) |
| Ancamine^{®} K54 | 2,4,6-Tris(dimethylaminomethyl)phenol (von Evonik) |

### Herstellung von Aminen:

### Reaktionsprodukt P-1: (enthaltend N-Furfuryl-1,2-ethandiamin; 1:1 Stöchiometrie)

In einem Rundkolben wurden 30.05 g (0.5 mol) 1,2-Ethandiamin unter Stickstoffatmosphäre bei Raumtemperatur vorgelegt. Unter gutem Rühren wurden 48.05 g (0.5 mol) Furfural (Furan-2-carbaldehyd, RCI = 1) zugegeben und 1 Stunde bei 40 °C nachgerührt. Die Reaktionsmischung wurde mit 1'000 ml Isopropanol versetzt und anschliessend bei einem Wasserstoff-Druck von 70 bar, einer Temperatur von 70 °C und einem Fluss von 5.5 ml/min auf einer kontinuierlich arbeitenden Hydrierapparatur mit Raney-Nickel-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,2-Ethandiamin, Wasser und Isopropanol entfernt wurden. Die so erhaltene Reaktionsmischung war eine klare, leicht gelbliche Flüssigkeit mit einer Aminzahl von 695 mg KOH/g, einer Viskosität bei 20 °C von 10 mPa·s und einem mittels GC bestimmten Gehalt an N-Furfuryl-1,2-ethandiamin von ca. 57.9 Gewichts-% (Retentionszeit 7.3 min), N-Tetrahydrofurfuryl-1,2-ethandiamin von ca. 8.2 Gewichts-% (Retentionszeit 8.0 min), N,N'-Difurfuryl-1,2-ethandiamin von ca. 31.6 Gewichts-% (Retentionszeit 11.9 min) und Anteile von am Furanring hydriertem N,N'-Difurfuryl-1,2-ethandiamin von ca. 2.3 Gewichts-% (Retentionszeit 12.5 min). Für die weitere Verwendung wurde mit einem AHEW von 58.9 g/eq gearbeitet.

### Reaktionsprodukt P-2: (enthaltend N-Furfuryl-1,2-ethandiamin; 2:1 Stöchiometrie)

In einem Rundkolben wurden 60.1 g (1 mol) 1,2-Ethandiamin unter Stickstoffatmosphäre bei Raumtemperatur vorgelegt. Unter gutem Rühren wurden 48.05 g (0.5 mol) Furfural (Furan-2-carbaldehyd, RCI = 1) zugegeben und 1 Stunde bei 40 °C nachgerührt. Die Reaktionsmischung wurde mit 1'000 ml Isopropanol versetzt und anschliessend bei einem Wasserstoff-Druck von 70 bar, einer Temperatur von 70 °C und einem Fluss von 5.5 ml/min auf einer kontinuierlich arbeitenden Hydrierapparatur mit Raney-Nickel-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,2-Ethadiamin, Wasser und Isopropanol entfernt wurden. Die so erhaltene Reaktionsmischung war eine klare, leicht gelbliche Flüssigkeit mit einer Aminzahl von 772 mg KOH/g, einer Viskosität bei 20 °C von 11 mPa·s und einem mittels GC bestimmten Gehalt an N-Furfuryl-1,2-ethandiamin von ca. 78.2 Gewichts-% (Retentionszeit 7.3 min), N-Tetrahydrofurfuryl-1,2-ethandiamin von ca. 12.3 Gewichts-% (Retentionszeit 8.0 min) und N,N'-Difurfuryl-1,2-ethandiamin von ca. 9.1 Gewichts-% (Retentionszeit 11.9 min). Für die weitere Verwendung wurde mit einem AHEW von 56.6 g/eq gearbeitet.

### Reaktionsprodukt P-3: (enthaltend N-Furfuryl-1,2-ethandiamin; 3:1 Stöchiometrie)

In einem Rundkolben wurden 60.1 g (1 mol) 1,2-Ethandiamin unter Stickstoffatmosphäre bei Raumtemperatur vorgelegt. Unter gutem Rühren wurden 32.0 g (0.33 mol) Furfural (Furan-2-carbaldehyd, RCI = 1) zugegeben und 1 Stunde bei 40 °C nachgerührt. Die Reaktionsmischung wurde mit 1'000 ml Isopropanol versetzt und anschliessend bei einem Wasserstoff-Druck von 65 bar, einer Temperatur von 65 °C und einem Fluss von 5.5 ml/min auf einer kontinuierlich arbeitenden Hydrierapparatur mit Raney-Nickel-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,2-Ethandiamin, Wasser und Isopropanol entfernt wurden. Die so erhaltene Reaktionsmischung war eine klare, leicht gelbliche Flüssigkeit mit einer Aminzahl von 757 mg KOH/g, einer Viskosität bei 20 °C von 10 mPa·s und einem mittels GC bestimmten Gehalt an N-Furfuryl-1,2-ethandiamin von ca. 86.1 Gewichts-% (Retentionszeit 7.3 min), N-Tetrahydrofurfuryl-1,2-ethandiamin von ca. 3.3 Gewichts-% (Retentionszeit 8.0 min), N,N'-Difurfuryl-1,2-ethandiamin von ca. 2.8 Gewichts-% (Retentionszeit 11.9 min), Anteile von am Furanring hydriertem N,N'-Difurfuryl-1,2-ethandiamin von ca. 1.5 Gewichts-% (Retentionszeit 12.5 min) und N,N,N'-Trisfurfuryl-1,2-ethandiamin von ca. 6.0 Gewichts-% (Retentionszeit 14.2 min). Für die weitere Verwendung wurde mit einem AHEW von 51.6 g/eq gearbeitet.

### Reaktionsprodukt P-4: (enthaltend N-Furfuryl-1,2-ethandiamin; 3.5:1 Stöchiometrie, Pd/C)

In einem Rundkolben wurden 105.2 g (1.75 mol) 1,2-Ethandiamin unter Stickstoffatmosphäre bei Raumtemperatur vorgelegt. Unter gutem Rühren wurde eine Lösung aus 48.05 g g (0.5 mol) Furfural (Furan-2-carbaldehyd, RCI = 1) in 200 ml Isopropanol zugegeben und 1 Stunde bei 40 °C nachgerührt. Die Reaktionsmischung wurde mit weiteren 1'000 ml Isopropanol versetzt und anschliessend bei einem Wasserstoff-Druck von 80 bar, einer Temperatur von 80 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,2-Ethandiamin, Wasser und Isopropanol entfernt wurden. Die so erhaltene Reaktionsmischung war eine klare, leicht gelbliche Flüssigkeit mit einer Aminzahl von 691 mg KOH/g, einer Viskosität bei 20 °C von 13.5 mPa·s und einem mittels GC bestimmten Gehalt an N-Furfuryl-1,2-ethandiamin von ca. 72.8 Gewichts-% (Retentionszeit 7.3 min), N-Tetrahydrofurfuryl-1,2-ethandiamin von ca. 7.8 Gewichts-% (Retentionszeit 8.0 min), N,N'-Difurfuryl-1,2-ethandiamin von ca. 3.3 Gewichts-% (Retentionszeit 11.9 min), Anteile von am Furanring hydriertem N,N'-Difurfuryl-1,2-ethandiamin von ca. 3.0 Gewichts-% (Retentionszeit 12.5 min), N,N,N'-Trisfurfuryl-1,2-ethandiamin von ca. 9.9 Gewichts-% (Retentionszeit 14.2 min) und Anteile von am Furanring hydriertem N,N,N'-Trisfurfuryl-1,2-ethandiamin von ca. 3.2 Gewichts-% (Retentionszeit ca. 14.7 min). Für die weitere Verwendung wurde mit einem AHEW von 55 g/eq gearbeitet.

### N-Furfuryl-1,2-ethandiamin (F-EDA):

41.2 g des Reaktionsprodukts **P-4,** hergestellt wie vorgängig beschrieben, wurden bei 70 °C unter Vakuum destilliert, wobei 25.6 g Destillat bei einer Dampftemperatur von ca. 50 °C und 0.1 bar aufgefangen wurden. Erhalten wurde eine farblose Flüssigkeit mit einer Aminzahl von 802 mg KOH/g, einem AHEW von etwa 46.7 g/eq, einem RCI von 0.71, einer Viskosität von 3.2 mPa·s bei 20 °C und einem mittels GC bestimmten Gehalt an N-Furfuryl-1,2-ethandiamin von 94.6 Gewichts-% (Retentionszeit 7.3 min) und N-Tetrahydrofurfuryl-1,2-ethandiamin von 5.3 Gewichts-% (Retentionszeit 8.0 min), welche im Folgenden als **F-EDA** eingesetzt wurde.

¹H-NMR (CDCl₃): 7.33 (d, 1 H, Ar-H), 6.27 (m, 1 H, Ar-H), 6.14 (m, 1 H, Ar-H), 3.76 (s, 2 H, Ar-CH₂), 2.78 (m, 2 H, NHC*H*₂CH₂), 2.65 (m, 2 H, C*H*₂NH₂), 1.52 (br s, 3 H, NH und NH₂).

FT-IR: 3284, 3043, 2945, 2838, 1567, 1504, 1455, 1382, 1306, 1219, 1146,1108, 1073, 1009, 916, 883, 806, 738.

### N,N'-Difurfuryl-1,2-ethandiamin (BisF-EDA)

In einem Rundkolben wurden 11.12 g (0.185 mol) 1,2-Ethandiamin in 200 ml Isopropanol unter Stickstoffatmosphäre bei Raumtemperatur vorgelegt. Unter gutem Rühren wurden 35.0 g g (0.37 mol) Furfural (Furan-2-carbaldehyd, RCI = 1) zugegeben und 1 Stunde bei 40 °C nachgerührt. Die Reaktionsmischung wurde mit weiteren 800 ml Isopropanol versetzt und anschliessend bei einem Wasserstoff-Druck von 65 bar, einer Temperatur von 65 °C und einem Fluss von 5.5 ml/min auf einer kontinuierlich arbeitenden Hydrierapparatur mit Raney-Nickel-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,2-Ethandiamin, Wasser und Isopropanol entfernt wurden. Erhalten wurde eine klare, leicht gelbliche Flüssigkeit, welche bei 110 bis 130 °C unter Vakuum destilliert wurde, wobei das Destillat bei einer Dampftemperatur von 105 bis 110 °C und 0.15 bar aufgefangen wurden. Erhalten wurde eine farblose Flüssigkeit mit einer Aminzahl von 502 mg KOH/g, einem AHEW von etwa 110 g/eq, einer Viskosität von 28 mPa·s bei 20 °C und einem mittels GC bestimmten Gehalt an N,N'-Difurfuryl-1,2-ethandiamin von 79.0 Gewichts-% (Retentionszeit 11.9 min), Anteile von am Furanring hydriertem N,N'-Difurfuryl-1,2-ethandiamin von ca. 11.9 Gewichts-% (Retentionszeit 12.4 bis 12.5 min), N,N,N'-Trisfurfuryl-1,2-ethandiamin von ca. 4.4 Gewichts-% (Retentionszeit 14.2 min) und Anteile von am Furanring hydriertem N,N,N'-Trisfurfuryl-1,2-ethandiamin von ca. 3.7 Gewichts-% (Retentionszeit ca. 14.6 bis 14.7 min), welche im Folgenden als **BisF-EDA** eingesetzt wurde.

### N-Tetrahydrofurfuryl-1,2-ethandiamin (THF-EDA):

In einem Rundkolben wurden 60.1 g (1 mol) 1,2-Ethandiamin unter Stickstoffatmosphäre bei Raumtemperatur vorgelegt. Unter gutem Rühren wurde langsam eine Lösung aus 32.0 g (0.33 mol) Furfural in 200 ml Isopropanol dazugetropft und 1 Stunde bei 40 °C nachgerührt. Die Reaktionsmischung wurde mit weiteren 300 ml Isopropanol versetzt und anschliessend bei einem Wasserstoff-Druck von 90 bar, einer Temperatur von 110 °C und einem Fluss von 5 ml/min auf einer kontinuierlich arbeitenden Hydrierapparatur mit Raney-Nickel-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,2-Ethandiamin, Wasser und Isopropanol entfernt wurden.

Erhalten wurde eine klare, leicht gelbliche Flüssigkeit, welche bei 70 °C unter Vakuum destilliert wurde, wobei 35.6 g Destillat bei einer Dampftemperatur von ca. 50 °C und 0.1 bar aufgefangen wurden. Erhalten wurde eine farblose Flüssigkeit mit einer Viskosität von 4.3 mPa·s bei 20 °C, einer Aminzahl von 728 mg KOH/g, einem AHEW von 48.1 g/eq und einem mittels GC bestimmten Gehalt an N-Tetrahydrofurfuryl-1,2-ethandiamin von 97 Gewichts-% (Retentionszeit 8.0 min), welche im Folgenden als **THF-EDA** eingesetzt wurde.

### N-Benzyl-1,2-ethandiamin (B-EDA):

180.3 g (3 mol) 1,2-Ethandiamin wurden bei Raumtemperatur vorgelegt, mit einer Lösung aus 106.0 g (1 mol) Benzaldehyd in 1200 ml Isopropanol vermischt, 2 Stunden gerührt und anschliessend bei 80°C, 80 bar Wasserstoff-Druck und einem Fluss von 5 ml/min auf einer kontinuierlich arbeitenden Hydrierapparatur mit Pd/C-Festbettkatalysator hydriert und die hydrierte Lösung am Rotationsverdampfer bei 65°C eingeengt, wobei unreagiertes 1,2-Ethandiamin, Wasser und Isopropanol entfernt wurden. Die so erhaltene Reaktionsmischung war eine klare, leicht gelbliche Flüssigkeit mit einem mittels GC bestimmten Gehalt an N-Benzyl-1,2-ethandiamin von ca. 81 Gewichts-% (Retentionszeit 8.5 min) und N,N'-Dibenzyl-1,2-ethandiamin von ca. 14 Gewichts-% (Retentionszeit 14.3 min). Sie wurde bei 80°C unter Vakuum mittels Destillation gereinigt. Erhalten wurde eine farblose Flüssigkeit mit einem AHEW von 50.1 g/eq und einem mittels GC bestimmten Gehalt an N-Benzyl-1,2-ethandiamin von > 97%, welche im Folgenden als **B-EDA** eingesetzt wurde.

### Herstellung von Addukten:

### Addukt A1:

51.3 g N-Furfuryl-1,2-ethandiamin (F-EDA, 0.366 mol) wurden auf 70 °C erwärmt und unter gutem Rühren langsam 45.0 g Araldite^{®} GY 250 (0.241 mol EP-Gruppen) zugegeben, wobei die Temperatur der Reaktionsmischung zwischen 70 und 90 °C gehalten wurde. Die Reaktionsmischung wurde während einer Stunde in diesem Temperaturbereich gerührt und anschliessend abgekühlt. Es wurde eine klare, leicht gelbliche Flüssigkeit mit einer Viskosität bei 20 °C von 124 Pa·s, einer Aminzahl von 419 mg KOH/g und einem berechneten AHEW von 112.3 g/eq erhalten.

### Addukt A2:

58.6 g N-Furfuryl-1,2-ethandiamin (F-EDA, 0.418 mol) wurden auf 70 °C erwärmt und unter gutem Rühren langsam 37.3 g D.E.N.^{®} 438 (0.208 mol EP-Gruppen) zugegeben, wobei die Temperatur der Reaktionsmischung zwischen 70 und 90 °C gehalten wurde. Die Reaktionsmischung wurde während einer Stunde in diesem Temperaturbereich gerührt und anschliessend abgekühlt. Es wurde eine klare, leicht gelbliche Flüssigkeit mit einer Viskosität bei 20 °C von 40.8 Pa·s, einer Aminzahl von 492 mg KOH/g und einem berechneten AHEW von 91.6 g/eq erhalten.

### Addukt A3 (Ref.):

55.0 g N-Benzyl-1,2-ethandiamin (B-EDA, 0.366 mol) wurden auf 70 °C erwärmt und unter gutem Rühren langsam 45.0 g Araldite^{®} GY 250 (0.241 mol EP-Gruppen) zugegeben, wobei die Temperatur der Reaktionsmischung zwischen 70 und 90 °C gehalten wurde. Die Reaktionsmischung wurde während einer Stunde in diesem Temperaturbereich belassen und anschliessend abgekühlt. Es wurde eine klare, leicht gelbliche Flüssigkeit mit einer Viskosität bei 20 °C von 262 Pa·s, einer Aminzahl von 408 mg KOH/g und einem berechneten AHEW von 116.3 g/eq erhalten.

### Herstellung von Epoxidharz-Zusammensetzungen:

### Beispiele Z-1 und Ref-1 bis Ref-3

Für jedes Beispiel wurden die in Tabelle 1 angegebene Harz- und Härter-Komponente separat auf eine Temperatur von 60 °C aufgewärmt. Mit diesen vorgewärmten Komponenten wurde dann eine Portion von insgesamt 20 g Epoxidharz-Zusammensetzung hergestellt, indem die Komponenten im in Tabelle 1 angegebenen Gewichtsverhältnis mittels eines Zentrifugalmischers (SpeedMixer^{™} DAC 150, FlackTek Inc.) während 15 Sekunden vermischt und dann unverzüglich folgendermassen geprüft wurden:

Die vermischte Zusammensetzung wurde in ein mittels Wasserbad auf 60 °C thermostatisiertes Reagenzglas gegeben und ein Thermofühler in der Mitte des vermischten Materials platziert. Damit wurde die Zeit bis zum Erreichen der maximalen Temperatur (in der Tabelle angegeben als **time to peak exotherm)** und die Höhe der maximalen Temperatur **(peak exotherm temperature)** im vermischten Material bestimmt. Die in der Tabelle 1 angegebenen Werte sind Mittelwerte aus drei Messungen.

Der **Tg**-Wert (Glasübergangstemperatur) wurde mittels DSC an ausgehärteten Proben aus der Mitte des Reagenzglases von der oben beschriebenen Bestimmung gemessen, wobei diese Proben vor der Messung zusätzlich während 14 Tagen im Normklima gelagert waren. Die Messung erfolgte mit einem Mettler Toledo DSC 3+ 700 Gerät und dem Messprogramm (1) -10 °C während 2 min, (2) -10 bis 200 °C mit einer Aufheizrate von 10 K/min (= 1st run), (3) 200 bis -10 °C mit einer Kühlrate von -50 K/min, (4) -10 °C während 2 min, (5) -10 bis 180 °C mit einer Aufheizrate von 10 K/min (= 2nd run).

Die Resultate sind in Tabelle 1 angegeben.

Bei der Epoxidharz-Zusammensetzungen **Z-1** handelt es sich um ein erfindungsgemässes Beispiel. Bei den Epoxidharz-Zusammensetzungen **Ref-1** bis **Ref-3** handelt es sich um Vergleichsbeispiele.

**Tabelle 1: Zusammensetzung und Eigenschaften von Z-1 und Ref-1 bis Ref-2.**

| **Beispiel** | | **Z-1** | **Ref-1** | **Ref-2** | **Ref-3** |
|---|---|---|---|---|---|
| **Harz-Komp.**: | | | | | |
| Araldite^{®} GY 250 | | 187.0 | 187.0 | 187.0 | 187.0 |

| **Härter-Komp.:** | | | | | |
|---|---|---|---|---|---|
| | **F-EDA** | 46.7 | - | - | - |
| | **B-EDA** | - | 50.1 | - | - |
| | MXDA | - | - | 34.0 | - |
| | IPDA | - | - | - | 42.6 |
| time to peak exotherm [min] | | 14 | 12 | 12 | 21 |
| peak exotherm temperature | | 106 °C | 201 °C | 150 °C | 74 °C |
| Tg 1st / 2nd run [°C] | | 50 / 90 | 53 / 88 | 93 / 100 | 76 / 155 |

### Beispiele Z-2 bis Z-11 und Ref-4 bis Ref-8

Für jedes Beispiel wurden die in den Tabellen 2 bis 4 angegebenen Inhaltsstoffe der Harz-Komponente in den angegebenen Mengen (in Gewichtsteilen) mittels eines Zentrifugalmischers (SpeedMixer^{™} DAC 150, FlackTek Inc.) vermischt und unter Ausschluss von Feuchtigkeit aufbewahrt.

Ebenso wurden die den Tabellen 2 bis 4 angegebenen Inhaltsstoffe der Härter-Komponente verarbeitet und aufbewahrt.

Anschliessend wurden die beiden Komponenten jeder Zusammensetzung mittels des Zentrifugalmischers zu einer homogenen Flüssigkeit verarbeitet und diese unverzüglich folgendermassen geprüft:
Die **Viskosität** wurde 5 min nach dem Vermischen der Harz- und der Härter-Komponente wie beschrieben bei einer Temperatur von 20 °C gemessen.

Die **Gelierzeit** wurde bestimmt, indem eine frisch vermischte Menge von ca. 3 g im Normklima mit einem Spatel in regelmässigen Abständen bewegt wurde, bis die Masse gelierte.

Die **Shore D** Härte wurde bestimmt nach DIN 53505 an zwei zylindrischen Prüfkörpern (Durchmesser 20 mm, Dicke 5 mm), wobei einer im Normklima und einer bei 8 °C und 80% relativer Feuchtigkeit gelagert und die Härte jeweils nach 1 Tag (24h) und nach 2 Tagen gemessen wurde.

Weiterhin wurde ein Film in einer Schichtdicke von 500 µm auf eine Glasplatte aufgezogen und dieser im Normklima gelagert bzw. ausgehärtet. An diesem Film wurde die **Königshärte** (Pendelhärte nach König nach DIN EN ISO 1522) nach 1 Tag, 2 Tagen, 7 Tagen und nach 14 Tagen bestimmt **(1d NK), (2d NK), (7d NK), (14d NK).** Nach 14 Tagen wurde der **Aspekt (NK)** des Films beurteilt. Als "schön" wurde ein klarer Film bezeichnet, welcher eine glänzende und nichtklebrige Oberfläche ohne Struktur aufwies. Als "Struktur" wird dabei jegliche Art von Zeichnung oder Muster auf der Oberfläche bezeichnet.

Ein weiterer Film wurde in einer Schichtdicke von 500 µm auf eine Glasplatte aufgezogen und dieser unmittelbar nach dem Applizieren während 7 Tagen bei 8 °C und 80% relativer Feuchtigkeit und anschliessend während 2 Wochen im Normklima gelagert, beziehungsweise ausgehärtet. 24 Stunden nach der Applikation wurde ein Flaschendeckel aus Polypropylen auf den Film aufgesetzt, unter welchem ein feuchter Schwamm platziert war. Nach weiteren 24 Stunden wurden der Schwamm und der Deckel entfernt und an einer neuen Stelle des Films platziert, wo sie nach 24 Stunden wieder entfernt und neu platziert wurden, insgesamt 4 mal. Anschliessend wurde der Aspekt dieses Films beurteilt (in den Tabellen mit **"Aspekt (8°/80%)"** bezeichnet), auf die gleiche Weise wie für den Aspekt (NK) beschrieben. Dabei wurde jeweils auch die Anzahl und Art der sichtbaren Marken angegeben, die im Film durch den feuchten Schwamm oder den aufgesetzten Deckel entstanden waren. Als "Blushing" wurde die Anzahl weiss verfärbte Flecken angegeben. Als "(1)" wurde ein schwacher, weiss verfärbter Fleck bezeichnet. Als "1" wurde ein deutlicher, weiss verfärbter Fleck bezeichnet. Als "Ring" wurde angegeben, ob ein ringförmiger Abdruck durch Einsinken des ersten, 24h nach Applikation aufgesetzten Deckels vorhanden war. Ein solcher ringförmiger Abdruck zeigt an, dass die Beschichtung noch nicht begehbar ist. An den so ausgehärteten Filmen wurde wiederum die Königshärte bestimmt, jeweils nach 7 Tagen bei 8 °C und 80% relativer Feuchtigkeit **(Königsh. (7d 8°/80%)),** dann nach weiteren 2 Tagen im NK **(Königsh. (+2d NK))** bzw. 7 Tagen im NK **(Königsh. (+7d NK))** bzw. 14d im NK **(Königsh. (+14d NK)).**

Die Resultate sind in den Tabellen 2 bis 4 angegeben.

Bei den Epoxidharz-Zusammensetzungen **Z-2** bis **Z-10** handelt es sich um erfindungsgemässe Beispiele. Bei den Epoxidharz-Zusammensetzungen **Ref-4** bis **Ref-8** handelt es sich um Vergleichsbeispiele.

**Tabelle 2: Zusammensetzung und Eigenschaften von Z-2 bis Z-4 und Ref-4 bis Ref-6. ¹ nicht messbar (zu weich)**

| **Beispiel** | | **Z-2** | **Ref-4** | **Ref-5** | **Z-3** | **Z-4** | **Ref-6** |
|---|---|---|---|---|---|---|---|
| **Harz-Komponente:** | | | | | | | |
| Araldite^{®} GY-250 | | 167.2 | 167.2 | 167.2 | 167.2 | 167.2 | 167.2 |
| Araldite^{®} DY-E | | 31.8 | 31.8 | 31.8 | 31.8 | 31.8 | 31.8 |

| **Härter-Komponente:** | | | | | | | |
|---|---|---|---|---|---|---|---|
| | **F-EDA** | 46.7 | - | - | - | - | - |
| | **THF-EDA** | - | 48.1 | - | - | - | - |
| | **B-EDA** | - | - | 50.1 | - | - | - |
| | Addukt **A1** | - | - | - | 112.3 | - | - |
| | Addukt **A2** | - | - | - | - | 91.6 | - |
| | Addukt **A3** | - | - | - | - | - | 116.3 |
| Viskosität (10') [Pa·s] | | 0.20 | 0.20 | 0.22 | 13.8 | 5.3 | 14.6 |
| Gelierzeit (h:min) | | 5:00 | >5:30 | 5:15 | 2:30 | 2:30 | 2:30 |
| Shore D | (1d NK) | 75 | 67 | 75 | 74 | 80 | 73 |
| | (2d NK) | 78 | 73 | 76 | 76 | 82 | 75 |
| Shore D | (1d 8°/80%) | n.m.¹ | n.m.¹ | n.m.¹ | 61 | 56 | 63 |
| | (2d 8°/80%) | 61 | 49 | 62 | 74 | 70 | 76 |
| Königshärte [s] | (1d NK) | 41 | 24 | 116 | 162 | 137 | 175 |
| | (2d NK) | 63 | 43 | 148 | 192 | 182 | 204 |
| | (7d NK) | 119 | 91 | 190 | 203 | 203 | |
| | (14d NK) | 167 | 111 | 197 | | | |
| Aspekt (NK) | | schön | schön | schön | schön | schön | schön |
| Königsh. [s] | (7d 8°/80%) | 12 | n.m.¹ | 29 | 50 | 35 | 109 |
| | (+2d NK) | 25 | n.m.¹ | 67 | 102 | 59 | 190 |
| | (+7d NK) | 52 | n.m.¹ | 90 | 164 | 83 | 195 |
| | (+14d NK) | 61 | 6 | 104 | 171 | 124 | 197 |
| Aspekt (8°/80%) | | schön | Struktur | schön | schön | schön | schön |
| Blushing | | 1 | 4 | (1) | (1) | (1) | kein |
| Ring | | 1 | 2 | kein | kein | kein | kein |

**Tabelle 3: Zusammensetzung und Eigenschaften von Z-5 bis Z-6 und Ref-7 bis Ref-8.**

| **Beispiel** | | **Z-5** | **Ref-7** | **Z-6** | **Ref-8** |
|---|---|---|---|---|---|
| **Harz-Komponente:** | | | | | |
| | Araldite^{®} GY-250 | 167.2 | 167.2 | 167.2 | 167.2 |
| | Araldite^{®} DY-E | 31.8 | 31.8 | 31.8 | 31.8 |

| **Härter-Komponente:** | | | | | |
|---|---|---|---|---|---|
| | **F-EDA** | 33.1 | - | 14.5 | - |
| | **B-EDA** | - | 35.1 | - | 15.0 |
| | Addukt **A1** | 33.1 | - | 33.1 | - |
| | Addukt **A3** | - | 35.1 | - | 35.1 |
| | IPDA | - | - | 17.0 | 17.0 |
| | Benzylalkohol | - | - | 20.0 | 20.0 |
| | Ancamine^{®} K54 | - | - | 2.0 | 2.0 |
| Viskosität (10') [Pa·s] | | 0.66 | 0.75 | 0.82 | 0.92 |
| Gelierzeit (h:min) | | 3:40 | 3:40 | 3:10 | 3:20 |
| Shore D | (1d NK) | 79 | 78 | 75 | 77 |
| | (2d NK) | 80 | 79 | 77 | 78 |
| Shore D | (1d 8°/80%) | 23 | 38 | 31 | 38 |
| | (2d 8°/80%) | 58 | 66 | 67 | 71 |
| Königshärte | (1d NK) | 94 | 136 | 55 | 66 |
| [s] | (2d NK) | 133 | 181 | 101 | 108 |
| | (7d NK) | 153 | 198 | 136 | 155 |
| | (14d NK) | 199 | 209 | 167 | 168 |
| Aspekt (NK) | | schön | schön | schön | schön |
| Königsh. | (7d 8°/80%) | 15 | 45 | 21 | 29 |
| [s] | (+2d NK) | 46 | 130 | 95 | 119 |
| | (+7d NK) | 64 | 146 | 119 | 153 |
| | (+14d NK) | 83 | 152 | 129 | 155 |
| Aspekt (8°/80%) | | schön | schön | schön | schön |
| Blushing | | (1) | (1) | (1) | (1) |
| Ring | | kein | kein | kein | kein |

**Tabelle 4: Zusammensetzung und Eigenschaften von Z-7 bis Z-11. ¹ nicht messbar (zu weich) ² nicht messbar (klebrig)**

| **Beispiel** | | **Z-7** | **Z-8** | **Z-9** | **Z-10** | **Z-11** |
|---|---|---|---|---|---|---|
| **Harz-Komponente:** | | | | | | |
| | Araldite^{®} GY-250 | 167.2 | 167.2 | 167.2 | 167.2 | 167.2 |
| | Araldite^{®} DY-E | 31.8 | 31.8 | 31.8 | 31.8 | 31.8 |

| **Härter-Komponente:** | | | | | | |
|---|---|---|---|---|---|---|
| | Reaktionsprodukt **P-1** | 58.9 | - | - | - | - |
| | Reaktionsprodukt **P-2** | - | 56.6 | - | - | - |
| | Reaktionsprodukt **P-3** | - | - | 51.6 | - | - |
| | Reaktionsprodukt **P-4** | - | - | - | 55.0 | - |
| | **BisF-EDA** | - | - | - | - | 110.0 |
| Viskosität (10') [Pa·s] | | 0.22 | 0.18 | 0.23 | 0.28 | 0.17 |
| Gelierzeit (h:min) | | 6:25 | 5:05 | 5:10 | > 7:00 | > 7:00 |
| Shore D | (1d NK) | 67 | 74 | 74 | 40 | n.m.¹ |
| | (2d NK) | 74 | 77 | 79 | 50 | n.m.¹ |
| Shore D | (1d 8°/80%) | n.m.¹ | n.m.¹ | n.m.¹ | n.m.¹ | n.m.¹ |
| | (2d 8°/80%) | 59 | 70 | 67 | 48 | n.m.¹ |
| Königshärte [s] | (1d NK) | 31 | 49 | 29 | 8 | n.m.2 |
| | (2d NK) | 60 | 56 | 39 | 15 | 3 |
| | (7d NK) | 81 | 96 | 77 | 84 | 8 |
| | (14d NK) | 113 | 118 | 102 | 96 | 8 |
| Aspekt (NK) | | schön | schön | schön | schön | schön |

## Patentansprüche

1. Verwendung eines Härters enthaltend mindestens ein Amin der Formel (I) zum Aushärten von Epoxidharzen, 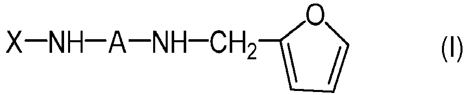 wobei A für einen linearen Alkylenrest mit 2 bis 10 C-Atomen steht und X für H oder Furfuryl steht.

2. Verwendung gemäss Anspruch 1, **dadurch gekennzeichnet, dass** A für einen Rest ausgewählt aus der Gruppe bestehend aus 1,2-Ethylen, 1,3-Propylen, 1,4-Butylen, 1,5-Pentylen und 1,6-Hexylen steht.

3. Verwendung gemäss einem der Ansprüche 1 oder 2, **dadurch gekennzeichnet, dass** A für 1,2-Ethylen steht.

4. Verwendung gemäss einem der Ansprüche 1 bis 3, **dadurch gekennzeichnet, dass** X für H steht.

5. Verwendung gemäss einem der Ansprüche 1 bis 4, **dadurch gekennzeichnet, dass** das Amin der Formel (I) einen RCI von mindestens 0.45, bevorzugt mindestens 0.6, insbesondere mindestens 0.7, am meisten bevorzugt von 1, aufweist, wobei RCI für das Verhältnis der Anzahl C-Atome aus biobasierter Quelle zur gesamten Anzahl C-Atome des Amins der Formel (I) steht.

6. Verwendung gemäss einem der Ansprüche 1 bis 5, **dadurch gekennzeichnet, dass** das Amin der Formel (I) in Form eines Reaktionsprodukts erhalten aus der reduktiven Alkylierung von mindestens einem Amin der Formel H₂N-A-NH₂ mit Furfural und Wasserstoff und nachfolgender Entfernung von unreagiertem Amin der Formel H₂N-A-NH₂ eingesetzt wird.

7. Verwendung gemäss Anspruch 6, **dadurch gekennzeichnet, dass** das Molverhältnis des Amins der Formel H₂N-A-NH₂ zu Furfural im Bereich von 1 bis 2, bevorzugt 1 bis 1.5, liegt.

8. Verwendung gemäss einem der Ansprüche 1 bis 7, **dadurch gekennzeichnet, dass** X für H steht und der Härter zusätzlich ein Amin der Formel 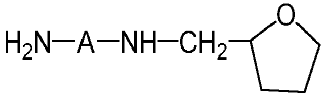 in einem Gewichtsverhältnis zwischen dem Amin der Formel (I) und dem Amin der Formel 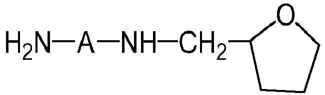 im Bereich von 70/30 bis 99/1, bevorzugt 80/20 bis 98/2, enthält.

9. Verwendung gemäss einem der Ansprüche 1 bis 8, **dadurch gekennzeichnet, dass** das Amin der Formel (I) teilweise oder vollständig in Form eines aminfunktionellen Addukts mit mindestens einem Epoxidharz oder Monoepoxid in einem stöchiometrischen Verhältnis von mindestens 1 mol Amin der Formel (I) auf 1 Mol-Equivalent Epoxidgruppen vorliegt.

10. Verwendung gemäss einem der Ansprüche 1 bis 9, **dadurch gekennzeichnet, dass** der Härter mindestens einen weiteren Bestandteil ausgewählt aus weiteren Aminen, welche nicht der Formel (I) entsprechen, Beschleunigern und Verdünnern enthält, insbesondere mindestens ein weiteres Amin, welches nicht der Formel (I) entspricht.

11. Verwendung gemäss Anspruch 10, **dadurch gekennzeichnet, dass** als weiteres Amin, welches nicht der Formel (I) entspricht, mindestens ein Amin mit einem RCI von 1 enthalten ist, insbesondere ausgewählt aus 2,5-Bis(aminomethyl)furan, 2,5-Bis(aminomethyl)tetrahydrofuran, Bis(5-aminomethylfuran-2-yl)methan, Bis(5-aminomethyltetrahydrofuran-2-yl)methan, 2,2-Bis(5-aminomethylfuran-2-yl)propan und 2,2-Bis(5-aminomethyltetrahydrofuran-2-yl)propan, wobei RCI für das Verhältnis der Anzahl C-Atome aus biobasierter Quelle zur gesamten Anzahl C-Atome des Amins steht.

12. Epoxidharz-Zusammensetzung umfassend
- eine Harz-Komponente umfassend mindestens ein Epoxidharz und
- eine Härter-Komponente umfassend den Härter wie in einem der Ansprüche 1 bis 11 beschrieben.

13. Ausgehärtete Epoxidharz-Zusammensetzung erhalten aus der Epoxidharz-Zusammensetzung gemäss Anspruch 12 nach dem Vermischen der Harz-Komponente und der Härter-Komponente.

14. Reaktionsprodukt erhalten aus der reduktiven Alkylierung eines Amins der Formel H₂N-A-NH₂ mit Furfural und Wasserstoff in einem Molverhältnis des Amins der Formel H₂N-A-NH₂ zu Furfural im Bereich von 1 bis 2, bevorzugt 1 bis 1.5, und nachfolgender Entfernung von Amin der Formel H₂N-A-NH₂ bis zu einem Gehalt von höchstens 1 Gewichts-%, bevorzugt höchstens 0.5 Gewichts-%, insbesondere höchstens 0.2 Gewichts-%, bezogen auf das Reaktionsprodukt, wobei A für einen linearen Alkylenrest mit 2 bis 10 C-Atomen, insbesondere für 1,2-Ethylen, steht.

15. Reaktionsprodukt gemäss Anspruch 14, **dadurch gekennzeichnet, dass** A für 1,2-Ethylen steht und das Reaktionsprodukt
50 bis 80 Gewichts-% N-Furfuryl-1,2-ethandiamin,
5 bis 50 Gewichts-%, insbesondere 5 bis 40 Gewichts-%, N,N'-Difurfuryl-1,2-ethandiamin,
0 bis 20 Gewichts-%, insbesondere 2 bis 15 Gewichts-%, N-Tetrahydrofurfuryl-1,2-ethandiamin,
weniger als 1 Gewichts-%, bevorzugt weniger als 0.5 Gewichts-%, insbesondere weniger als 0.2 Gewichts-%, 1,2-Ethandiamin und gegebenenfalls weitere Bestandteile, insbesondere weitere Nebenprodukte aus der reduktiven Alkylierung, enthält bezogen auf das Reaktionsprodukt.

16. Amingemisch enthaltend mindestens ein Amin der Formel 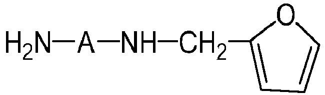 und mindestens ein Amin der Formel 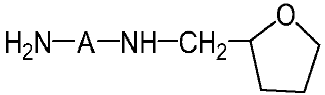 in einem Gewichtsverhältnis im Bereich von 70/30 bis 99/1, bevorzugt 80/20 bis 98/2, wobei A für einen linearen Alkylenrest mit 2 bis 10 C-Atomen, insbesondere für 1,2-Ethylen, steht.
